# EUROPEAN PATENT SPECIFICATION

(11) **EP 1 223 853 B1**
(45) Date of publication and mention of the grant of the patent: **27.09.2017**
(21) Application number: 00968822.7
(22) Date of filing: 06.10.2000
(51) Int. Cl.: A61B 5/00, G06F 9/00, A61B 8/00, G02B 21/36

(54) **VIRTUAL TELEMICROSCOPE**
VIRTUELLES TELEMIKROSKOP
TELEMICROSCOPE VIRTUEL

(30) Priority: 08.10.1999 US 158326 P
(43) Date of publication of application: 24.07.2002
(73) Proprietor: THE RESEARCH FOUNDATION OF STATE UNIVERSITY OF NEW YORK, Albany, New York 12201-0009 (US)
(72) Inventor: GU, Jiang, Mobile, AL 36608 (US); ANDERSON, Virginia, M., Rumson, NJ 07760 (US)
(74) Representative: Schnappauf, Georg
(86) International application number: PCT/US2000/027681
(87) International publication number: WO 2001/026541

(56) References cited:
- WO-A-98/01999
- WO-A-99/30264
- US-A- 5 542 003
- US-A- 5 891 035
- US-A- 5 933 519
- US-A- 5 954 650
- PATENT ABSTRACTS OF JAPAN vol. 1999, no. 10, 31 August 1999 (1999-08-31) & JP 11 133311 A (NIKON CORP), 21 May 1999 (1999-05-21)

## Description

Attached to this application as Appendix A is a print-out of software code applicable to the embodiments of the invention described herein.

### BACKGROUND OF THE INVENTION

This invention is related to a "Virtual Telemicroscope" (VT) system and, more particularly, to a method and system for using a computer system as a telemicroscope.

Telepathology is a field that combines the disciplines of pathology, computer science and telecommunication. It captures, digitizes, transmits, displays and manipulates pathological and medical images for the purpose of analysis, consultation, collaboration, diagnosis, training and standardization. Compared with conventional pathology, telepathology is more efficient, economical and flexible. It enables medical image evaluation to be performed at any location, any time, as long as the evaluator has access to a computer with adequate network connection.

The history of telemedicine and telepathology goes back to the early days of computer science and telecommunication, and its progress has paralleled the advancement of these two fields. Physicians have long been experimenting with the idea of delivering medical service to distant locations by means of telecommunication.

Telemedicine has come a long way. Certain disciplines, such as teleradiology, have been successfully implemented in many hospitals throughout the world. However, telepathology and telemedicine face a number of obstacles. These include computer speed and capacity, programming techniques, compression strategy, network transmission bandwidth, the way the images are displayed and manipulated, physicians' and technicians' training, as well as administrative issues such as medical licensing, legality, payment, medical insurance, patient privacy, etc.

In addition, for telepathology in particular, much of the resistance to these new technologies from the pathology community has been that the microscopic images have been handled and viewed in a way that is very different from the traditional manner in which the cases are evaluated. The transmitted images are usually static, isolated, and often represent only portions of the entire tissue section. The pathologists frequently hesitate in making any pathologic diagnosis based on the computer images of a partial tissue section displayed in an unfamiliar manner. When the entire specimen is digitized, the process takes a considerable amount of time (a few hours), involving specially designed and expensive automatic microscopic equipment and lengthy scanning steps. This, together with the limited computer capacity, the relatively narrow transmission bandwidth and the very high cost, has hindered the practical value of this potentially very useful technology up to the present day.

With the recent development of fast computers, wide band transmission network and new programming technology, this situation is rapidly changing. This VT system takes advantage of recent advancements and overcomes some of the major technical obstacles in telepathology. It aims to popularize this technology with a new approach.

In an international conference on telepathology on December 3-5, 1996, leading experts in the field of telepathology agreed that at sufficient resolution, the digitized computer images can be adequate for pathological diagnosis and consultation. There are systems for similar purposes. However, no practical system was available to provide a pathologist with a full magnified or non-magnified view of the entire specimen and at the same time a desired view of selected portions of the specimen at a different magnification. The available systems rely on remotely controlled microscope and camera and broadband network connection, or lengthy process of digitization of slides, involving cost far beyond the justification of the practical value for most pathologists worldwide.

### SUMMARY OF THE INVENTION

In accordance with the invention, there is provided a method and a system of using a computer system as a telemicroscope. Among the exemplary aspect of the invention, a first image corresponding to an area of a specimen is captured with a digital image capturing device; at least one second image corresponding to a selected area of the first image is captured with the digital camera, said second image having a different magnification from the first image; the first and second images are stored in a computer-readable medium; and a linking information map is generated indicating the relationship between said first and second images.

In accordance with another exemplary aspect of the invention, a location for an index file is received from a user, wherein the index file is stored on a computer-readable medium; the index file is retrieved, wherein the index file lists a plurality of image files having an image of a specimen and map information of linked images; the listing of the plurality of image files from the index file are displayed on the monitor screen of the user's computer system; a first file name comprising an image of the entire specimen is received from the user, wherein the first file name is linked to a second file name comprising an image of a selected area of the specimen, wherein said image of said second file name has a different magnification level from the image in said first file name; and the images of said first and second file name are dynamically displayed allowing a user to view the specimen with different magnification levels of the specimen.

In accordance with yet another exemplary aspect of the invention, a plurality of images of a specimen are captured. The images correspond to an area of the specimen and one or more segments of the specimen. The images corresponding to the specimen segments have different magnification levels; a linking map is generated between said segment images. The linking map comprises information regarding geographical location of the images in relation to the specimen's structure; and such images and said linking map are transmitted to a remote user via a computer network, thereby allowing the user to view the specimen image in their entirety and at different areas of the entire images with different magnification levels.

For a complete understanding of the invention, together with its features, details and advantages, reference should be made to the following description of preferred embodiments and drawings.

### BRIEF DESCRIPTION OF THE DRAWINGS

Fig.1 is a diagram of the functional structure of the VT system according to the present invention;
Fig. 2(a) is a flowchart illustrating the major steps in image arrangement, packaging and posting;
Fig. 2(b) is a flow chart illustrating the major steps in image retrieval, reviewing and manipulation;
Fig. 3 is a pictorial representations of the hardware set up of a VT system showing the process of image acquisition using a high-resolution scanner, a light microscope with a digital camera and a computer;
Figs. 4a, 4b are screen views of image arrangement, linkage, and packaging. The image selection view (Fig. 4a). The image link and arrangement view (Fig. 4b);
Fig. 5 is a screen view of image retrieval;
Fig. 6 is a screen view of the automatically hidden toolbar and its icons;
Fig. 7 is a screen view of the magnifying glass effect;
Fig. 8 is a screen view of the microscope effect;
Fig. 9 is a screen view of the linked image map;
Fig. 10 is a screen view of the measurement function;
Figs. 11a-11j are flowcharts of the major functions of the VT program;
Fig. 12a is a printout of internal cast members where most of the casts are stored; and
Figs. 12b-12k are the layout of backstage scores where the design and arrangement of the sprites are illustrated.

### DETAILED DESCRIPTION OF THE INVENTION

### 1. Basic Principles

The present invention is a new "Virtual Telemicroscope (VT) system", in which images are captured, digitized, arranged, packaged, posted, transmitted, displayed, enlarged, measured and analyzed with a user-friendly software program. It can be used for telepathology, telemedicine, distance learning, remote training, standardized exam and other applications, in which high-resolution images are transmitted and evaluated. This invention enables the users to retrieve and view virtual slides with specimen images and logically linked high-resolution partial images anywhere, any time via the Internet and other computer networks, without involving special and expensive equipment and setup.

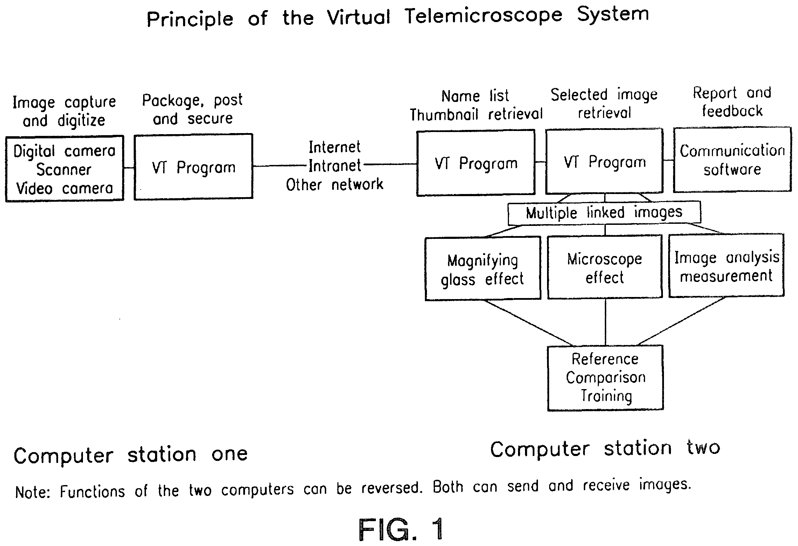

Fig. 1 shows a functional structure of the VT system according to the present invention, which can be used to create, retrieve and view virtual slides. The basic principle of this system closely mimics that of a light microscope. When a pathologist exams a specimen on a glass slide under a light microscope, he uses a number of objective lens with different magnifications. Each slide is viewed through a fixed magnification. By moving the glass slide on a movable stage, the entire slide is examined.

He usually views the slide with a low-power objective lens first to exam the entire specimen and then switches to a higher-power objective lens to have a closer look at different areas of interests. Essentially, he views static images in a dynamic manner. The VT system works the same way. It captures the image of a entire slide with a high-resolution scanner. Alternatively, a low-power objective lens connected with a digital image capturing device such as digital camera can be used for the image of the entire slide. Subsequently, high-power images are captured for selected areas with several high-power resolution lens with different magnification also connected with the digital image capturing device. The captured images are converted into a digitized images and stored. A link map information is then generated to link the image of the entire slide to the higher-power images. All these images, information concerning their location relative to specimen and magnifications, together with associated text data are then packaged and transmitted to a remote computer accessible by a user.

The remote computer displays the entire slide as a first image and a higher-power close-up views as a second image, as well as an image map that indicates the relationship between the first and second images. The relationship can be one or more of the degree of magnifications, size of the specimen represented by the image location of the selected area of the specimen from which the second image was prepared. The second image can be prepared as many as possible with different selected area and magnifications. The viewer can view different portions of the digitized images and enlarge them to a range of magnifications without loosing any clarity.

In summary, static images of different magnification lens and of corresponding different areas or sections from the same specimen are captured, arranged and packaged. They are then transmitted and viewed by a remote computer in a dynamic way, i.e. the viewers can examine the entire slide and then switch to selected higher-power images for a close-up evaluation. The viewer can also navigate the "virtual" slide in a way very similar to operating a light microscope. In addition, the VT system can perform image measurement, comparison, annotation, etc. and provide functions beyond those provided with a light microscope.

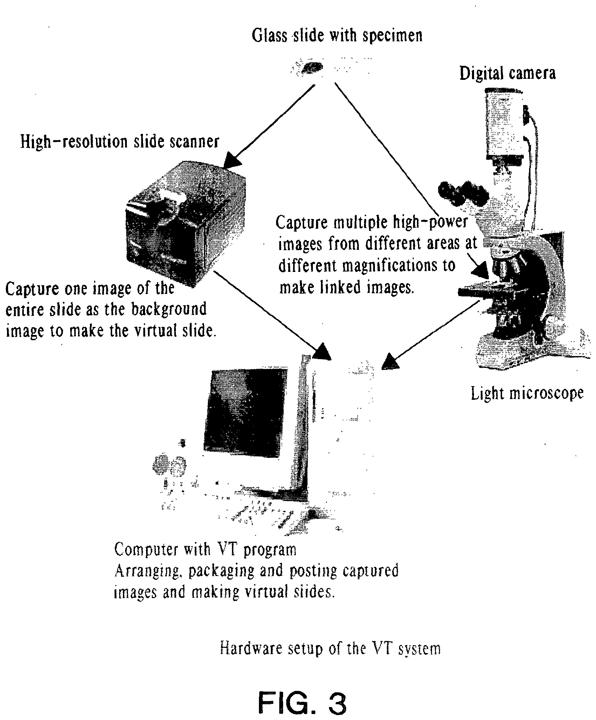

Fig. 3 shows pictorially a representative hardware set-up of the VT system. As an example, the following equipment is used; (1) a 166 MHz Pentium II or above class computer with at least 128 MB of RAM installed memory (384 MB recommended), 100 MB of available hard disk space, CD-ROM drive, Color monitor capable of 800 x 600 resolution (19 inch recommended), Windows 95, 98, NT or 2000, Internet or Intranet connection (broadband connection recommended); (2) a digital camera for a light microscope capable of capturing images of at least 800 x 600 pixels resolution (1152 x 864 pixels or higher resolution recommended); (3) a high-resolution slide scanner that can scan standard pathological glass slides at 2700 x 2700 dpi (4000 x 4000 dpi or higher recommended).

Fig. 3 illustrates the process of image acquisition using a high-resolution scanner, a light microscope with a digital camera and a computer. An image of the entire specimen is first captured with a high-resolution slide scanner or a light microscope using an objective lens at a very low magnification. This image of the entire specimen slide is used as the background image of the virtual slide. Higher-power images are then captured with the microscope and the digital camera from different areas of the same specimen using different objective lenses. All acquired images are saved in a folder containing logically-related image data in a computer. The VT program packages them into a virtual slide for transmission and viewing.

The VT system can be divided into the following six operational stages: acquire images, package images, transmit and retrieve images, display images, manipulate images and provide information feedback. Physically, the VT system comprises a memory unit and processing unit. The processing unit is in communication with the memory unit and is configured by the processor to perform such functions as capturing and storing images, generating and storing a link map between the images and transmitting the stored information to other users at remote computers through computer network.

As noted above, an image of the entire specimen on the glass slide is captured with a high-resolution scanner or, alternatively a digital camera and light microscope equipped with a low-power objective lens. This image is usually very large in size, much more than the usual computer screen size (800 x 600 pixels). Then, areas of interests from the same glass slide will be captured with a digital camera linked to a light microscope equipped with higher-power objective lenses. Preferably, additional images will be taken from different specimen areas at different magnifications from the same glass slide. All these images are captured in sizes equal to or larger than 800 x 600 pixels and saved in JPG format into the data file folder in the computer. Additional slides can be prepared in the same manner.

The VT program packages these saved images for transmission or posting. This is achieved by using the first image (the image of the entire specimen) as a background map and arranging all the other images taken from the same slide against this background. These images (as thumbnails) are digitally placed on the background map and positioned at the locations where they were captured. Their sizes will also be digitally adjusted to reflect their magnifications, i.e., the higher the magnification, the smaller the area they will cover on the background map.

Information including the images themselves, the locations and the image sizes, together with associated text data is saved and packaged into an index file. At this point, the VT program makes small thumbnail images of all the large images. The image files and the index file will then be posted on a computer server or sent to the receiver via email attachment. This completes the task for image preparation at the sending side of the system.

On the receiving side, a remote viewers will open up the VT program on his/her computer system and enter the complete address where the packaged images are posted. By clicking the "retrieving" button or the "enter" key, the VT program will retrieve the file names of the saved images and link information map. The retrieved files are shown as thumbnail images on the screen allowing a user to easily select an image out of several images shown. A thumbnail image on the screen represent a "slide tray" and the screen may can show up to 20 virtual slides simultaneously in an embodiment. The user can then see all the available images as virtual slides on the monitor screen. If the image package is transmitted to the remote computer via an e-mail attachment, the viewer will only need to locate the file on his computer. Once the file name is selected, the packaged images will be called it into the VT program for display.

The user picks any slide displayed on the screen for review by clicking on it. The image of the entire specimen (the one captured with the scanner or the low-power objective lens of the microscope connected with digital camera) will then be downloaded into the user's computer and displayed on the screen. The real size of the image (e.g., 2000 x 2000 pixel) is much larger than the monitor screen (800 x 600 pixels). At this point, the program shrinks the large image to fit into the size of the screen so that the reviewer can have a complete view of the entire specimen.

At this point, a viewer has a number of options. The viewer can choose one of the many functions built into the VT system, including evaluating the image with the "magnifying glass function", the "microscope function", the "measurement function", "retrieving text data" associated with the images, and retrieve additional high-power linked images to have a closer look of selected areas of interests from the same specimen. The viewer may also go back to the "virtual slide tray" to select another slide to view, as if working at an office desk with a slide tray and a light microscope. All such functions can be activated by "clicking" on corresponding icons in an automatically hidden toolbar, which is located at the upper margin of the screen.

The principle of the enlargement functions of the VT system is based on the fact that the original sizes of the captured images are very large, much larger than that of the screen size (800 x 600 pixels). The VT program manipulates the image to show only a portion of it at different ratios of sizes from the original image, and gives the viewers the impression of image enlargement. This is why the enlarged images are always sharp. The degree of enlargement is determined by the original size of the captured image.

In the magnifying glass effect, only a portion of the enlarged image is shown, mimicking the effect of a magnifying glass. In the microscope mode, a small portion of the virtual slide, as defined by a virtual objective lens, is displayed on the entire screen as the viewfinder, closely mimicking the effect of a light microscope.

A viewer can also click an icon to view the availability of the linked higher power images, together with their relative locations and sizes. By clicking on these linked images on the image map, the viewers can selectively evaluate these linked higher power images to have a better appreciation of the detailed structure of the specimen.

By selecting different icons in the toolbar, viewers can also perform line measurement of images, view associated text files, compare one image with other images in an established image database, and view the help file. The viewers can exit the program by clicking the "quit" button at any time.

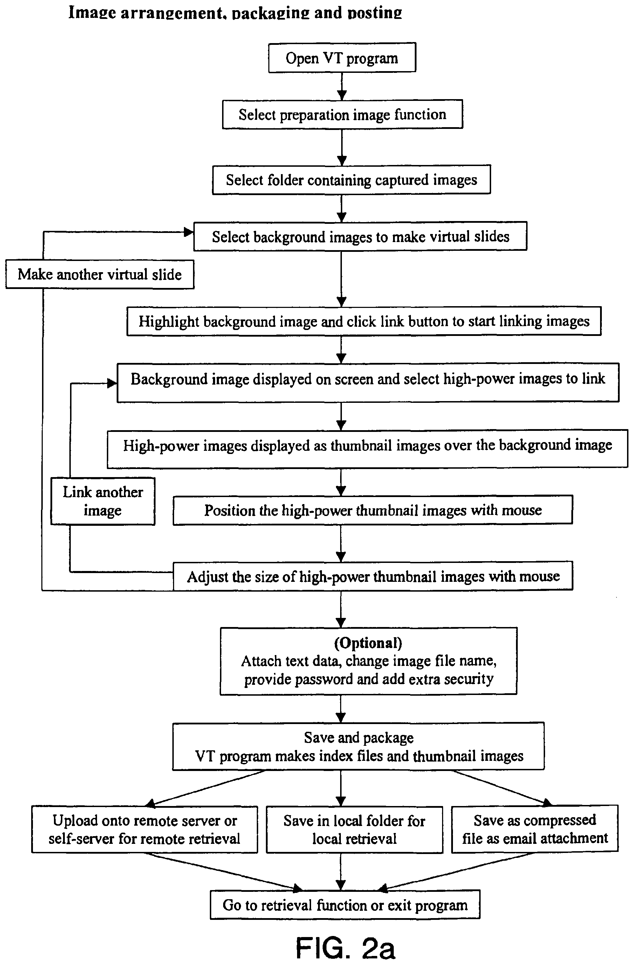

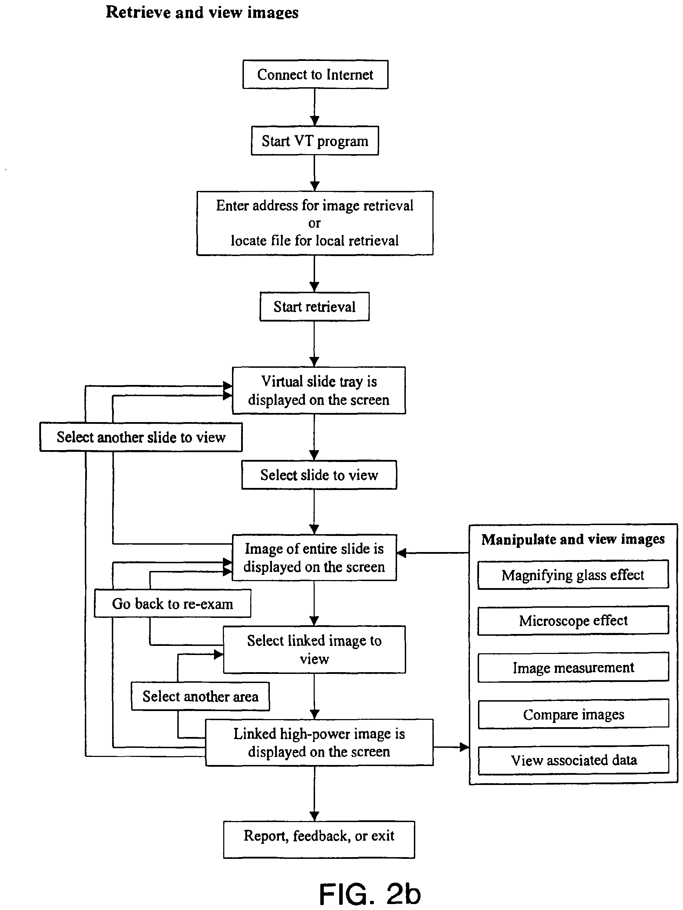

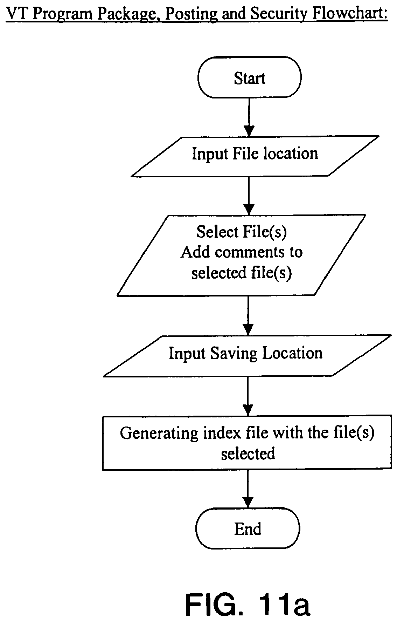

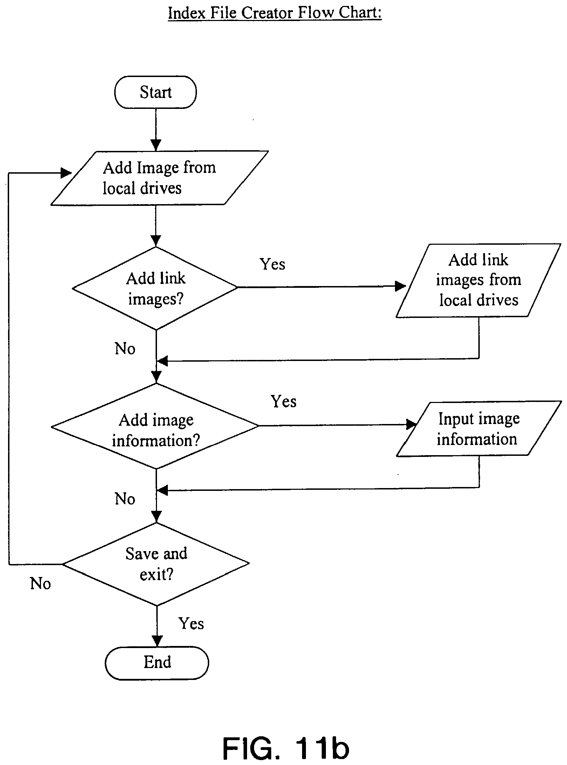

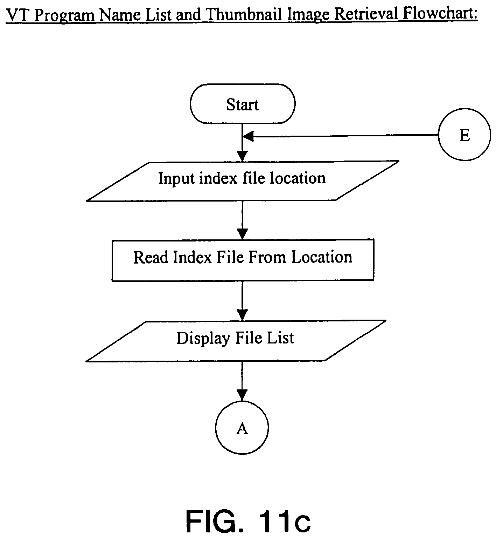

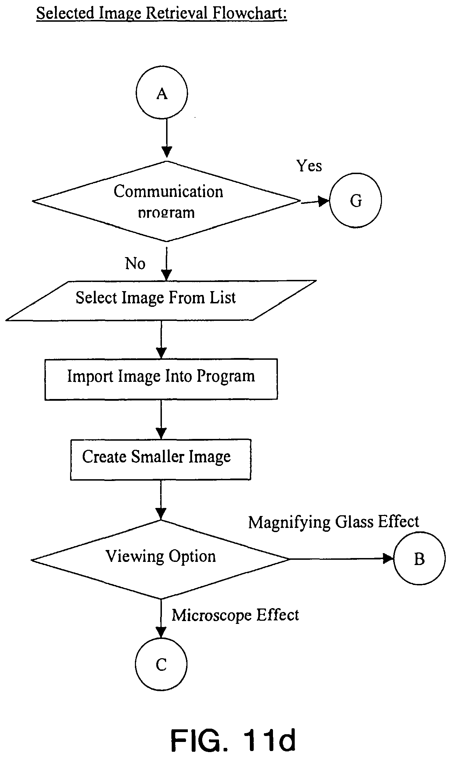

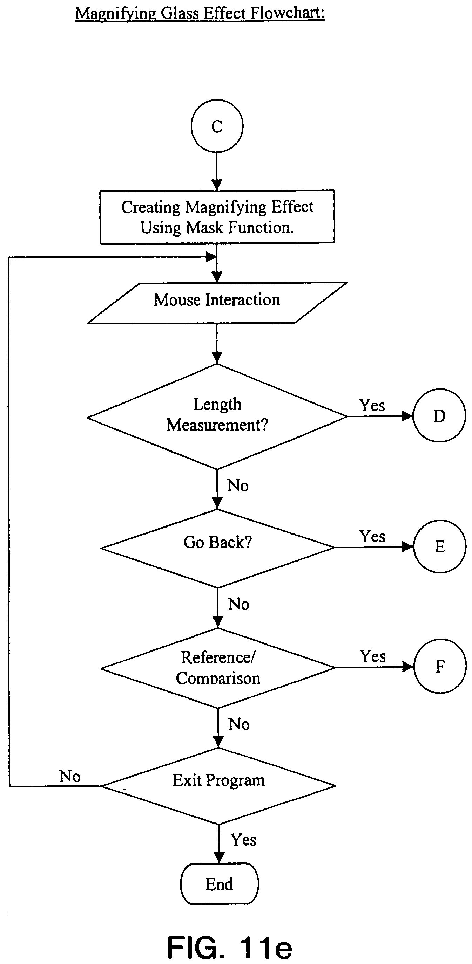

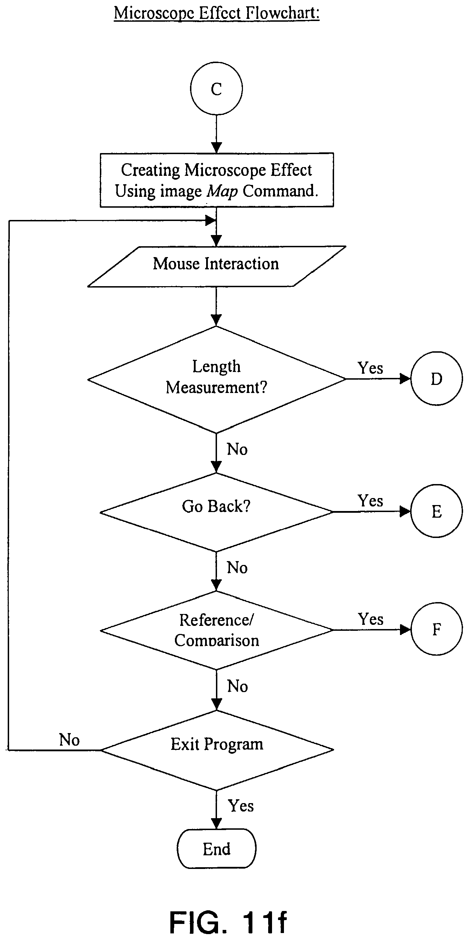

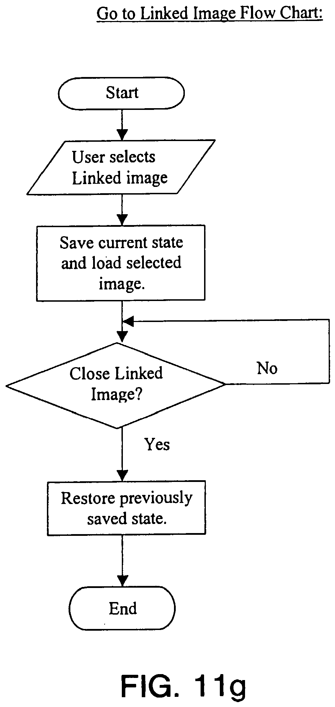

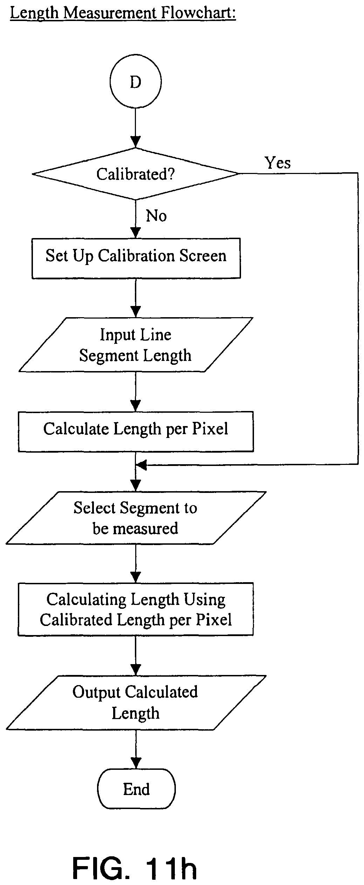

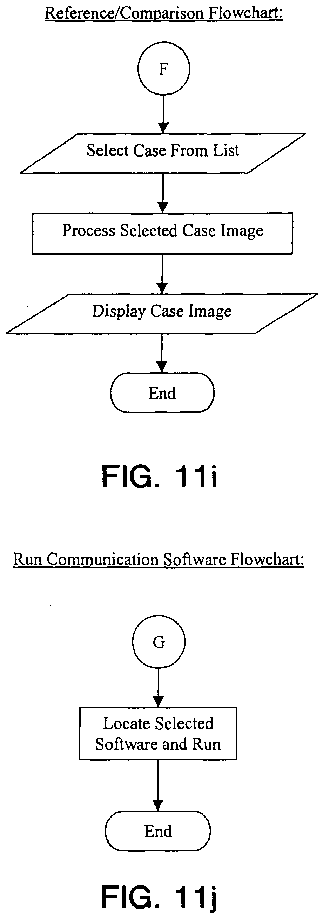

The basic functional organization of the VT system is illustrated in Fig. 1. The different steps involved in operating of the VT system is presented in Figs. 2a, 2b. The logical steps of the computer program are presented in the flow chart of Fig. 11. Details of each aspect of the system are described below.

### 2. Image acquisition

A pathological tissue section is first converted into a digital image by capturing with a digital microscopic camera or a high-resolution scanner. In either case, the entire pathological image can be digitized. The basic functional relationship among the slide scanner, the digital camera and computer in capturing the images is illustrated in Figure 3.

Depending on the objective lens used on the microscope, the entire image may be captured with one exposure or with a series of exposures of the tissue section and then a complete image formed by pasting multiple images together with software to make a seamless mosaic image. The low-power overview image also can be obtained with microscopic objectives at very low magnification.

Generally, it is easier to capture the image of the entire specimen with a high-resolution scanner. Currently, the highest resolution for a small area glass slide scanner is about 4000 x 4000 dpi. For a 0.5 x 0.5 inch tissue section, this will generate an image of about 2000 x 2000 pixels in size. When displayed on a computer monitor at 800 x 600 pixels with a 19-inch diagonal displaying area, this represents a real enlargement of the original sample at about 100-150 times.

On a computer monitor with a display capacity of 0.26-0.28 mm resolution, images at their real size can be further stretched 1.5 times without losing any visible resolution. When stretched beyond this magnitude, the quality of the image begins to deteriorate, and this may affect the accuracy of the pathologists' evaluation of the images. Therefore, with a high-resolution scanner, a tissue section can be effectively enlarged up to about 200 times from the original size without any visible distortion of the original image. This would magnify a typical human neutrophil (originally at about 15 um in diameter) to about 3-4 mm in diameter, enough to display microscopic patterns of cellular arrangement for most pathological cases. Slide scanner at other resolutions can also be used. However, the final size of the captured image preferably should be equal to or larger than 800x600 pixels.

Once the entire specimen is captured, one can further capture a number of high-resolution images from different areas of interest from the same slide using microscopic objective lens of higher magnifications. Each image should have the resolution of at least 800x600 pixels, preferably higher. These images can be arranged, linked and packaged together with the low-power image captured previously.

They will be transmitted collectively and viewed at the receiving end in a meaningful way to make sense of each high-power image in relation to each other and to the low-power image as the background. This will greatly enhance the effectiveness of image viewing by the remote computer. At the same time, this approach will save the user from capturing the entire slide with high-power objective lens, which consumes time and storage space and slows the entire process to an impractical level.

Once captured, the digital images can be saved into jpg, gif, tif or bmp file format. Depending on the file format, the file size of each high-resolution image varies, from less than one megabyte to several megabytes. To facilitate fast network transmission, it is recommended that the images be saved as jpg files with about 50 % compression. We have determined, through experiments and consultation with other pathologists, that at this rate of compression, there is no noticeable loss of image clarity. Further compression may be possible for different types of files. These images can be stored in any folder and drive, portable or fixed, in a computer for further packaging, posting and transmission.

### 3. Image preparation, packaging and posting

The captured images need to be arranged, packaged and saved into designated folders. Fig. 2a shows the major steps of image arrangement, packaging and posting. These packaged and saved files can be posted in a server (on a remote computer or the same computer) for remote retrieval, or in the same computer for local retrieval. The packaged images can also be compressed and attached with an email and sent to any email addresses. The VT program installed in a remote computer can open the email-attached files. This approach will bypass the need to have a computer server at the sending end and make it available to most pathologists and users. The viewing functions of the VT program are the same no matter how the image package is transmitted or retrieved.

If a server is used, the package is posted in certain designated folder that will make the packaged images available for retrieval by the Virtual Telemicroscope program located in the same or remote computers. The remote computers may be stationed across rooms, across nations or across continents, and connected by a network (local area network, Intranet or Internet). One set of packaged images can be retrieved and viewed by unlimited number of computers simultaneously. This feature facilitates online learning, online examination, collaboration, consultation, discussion and standardization.

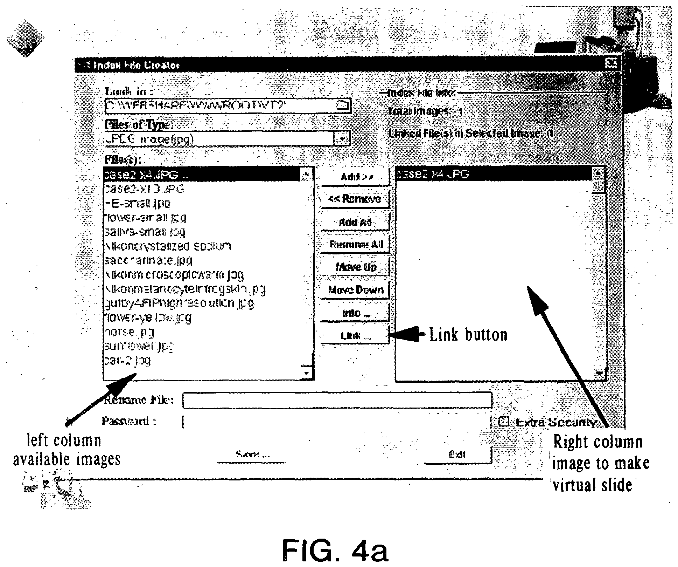

The packaging function is achieved by grouping and arranging all the images captured from the same glass slide together and record their locations and magnifications. By clicking on the "prepare image" button, a dialogue window of file directory is opened with two columns and a number of function buttons at the middle as shown in Fig. 4a. The image selection window contains two columns with function buttons in the middle. The left column displays available images for packaging. The right column displays images to be made into virtual slide.

The users can navigate through the file directory and identify the previously captured images. Once the folder is selected, file names of all the relevant image files will displayed in the left column. The users can then select the image files to be used as the background images (the ones captures with the slide scanner or the low power objective lens) and add them to the right column for further packaging and processing.

The images of up to 20 different slides can be comfortably processed and presented in a virtual slide tray for the viewer to view. Each of the twenty images can be linked and packaged to additional 20 higher power images taken from the same slide to give the viewers the option to have a closer exam of selected areas at higher magnifications. This linking and packaging function of the VT system is described below.

A unique feature of this system is that it can arrange a number of images at different magnifications taken from different areas of the same tissue sample and relate them to a background low magnification image in a way that the geographic relationship among the different images and their relative magnifications are recorded, transmitted and displayed.

This feature enables the viewers to use a low-power image of the entire slide as the background and link up to 20 higher-power images (this number can be increased if needed) of different areas to the same background. Each linked image can be positioned and sized on the background to reflect the location and area from where it is taken. This allows the senders and the viewers to appreciate the relationship among the different images taken from the same slide and greatly enhance the easiness for the reviewers to view and analyze the entire specimen.

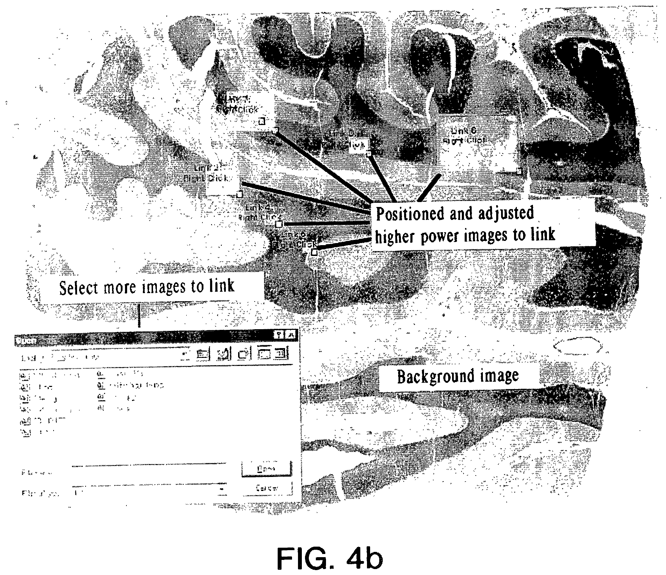

The linkage and package of multiple images taken from the same glass slide is achieved as following. First the file name of the background image is selected and then click on the button of "link". The selected background image is then displayed on the screen. At this time another file directory window is opened and the users will select the image files to be linked to the background image. When all the higher-power images to be linked to the background image are selected, they will be displayed at the margin of the background image as small thumbnail images as shown in Fig. 4b. The image linkage window contains the entire background image and many higher power images to be linked to the background image. These higher power images are displayed as smaller thumbnail images over the background and their positions can be moved and sizes readjusted with the mouse by the user to create a linked image package within the virtual slide. Their positions and sizes should correlate to the areas from which they were captured and the ranges of sizes they cover. All these information and the images will be packaged and transmitted. They can then be displayed faithfully in remote computers using the VT program.

The users can then drag and drop each image onto the background and position it to the appropriate location where it was captured. The users will then resize the images by dragging one corner of the linked images and shrink or enlarge them to appropriate sizes in relation to the background image, covering an area equivalent to the viewfinder sizes of the objective lens used in capturing these images. All the linked images are arranged against the background image in the same fashion. The entire package will then be saved. All the images, together with their locations and sizes and relationship to each other, will be recorded in the index file and transmitted to the remote computer for display.

During image packaging, the VT program also offer the possibility for the user to annotate the images by putting arrows, circles, rectangles, lines and words on the images. These marking can be recorded and transmitted to the retrieving computer and displayed by clicking an icon in the toolbar.

During image preparation and posting, the Virtual Telemicroscope program makes copies of the original digitized images and stores them in the designated folder on the server (a remote server or the local computer if it is used as its own server). Simultaneously, the VT program automatically creates an index file that compiles the file names or given names for each image. This file is saved as a Director (Macromedia Inc. San Francisco, CA, USA) cast file with an "cct", "cst" or "txt" extension. The former file format is protected that can not be opened or modified by a third party.

The VT program will also make thumbnail image from each high-resolution image and store them with the index file for retrieval. During image retrieval, the remote computer can access the designated folder to read this particular index file using the VT program, and display the available images on the remote computer. The images are first displayed as thumbnail images with complete file path and names in a virtual slide tray, resembling the glass slide tray used in routine pathology laboratories. One virtual slide tray can hold up to 20 slides. The viewer can retrieve slide trays one at a time and there is no limit to how many slide tray can be posted and retrieved. These slide trays can also be retrieved and displayed by the same computer where the files are stored. All these functions are performed in the background and are transparent to the users. All the viewers have to do is to enter the path or URL of the folder location, with a click of a button (the retrieval button), or a press of a key (the enter key); the virtual slides will be displayed on the monitor screen almost instantly.

When the packaged images are stored in the same computer, or transmitted as an email attachment, the user will first need to locate these files by clicking the "open file" icon next to the location window. A file directory window will open and allow the user to identify the packaged files. Once selected, the packaged images will be displayed in the screen in the same way as for the retrieved image packages.

The dialogue window for image preparation and posting also calls for optional attachment of data files of related information concerning the image. These can be patients' information, notes from the sender, specific requests, etc. Notes can be entered and files can be attached at this time. They will be stored under the same names as the image files but with different file extensions. These files are retrieved together with their linked image files.

In the dialogue window during image posting, there is also an entry for a password. The users have the option to enter a password to prevent the images from being accessed by unauthorized parties. If the users choose to leave the password window blank, no password will be required when retrieving images.

There is also an option named "extra security". If the use checks this option during image packaging, the images and data will be encrypted to provide extra protection to third party tampering. This option may slow down the speed at which the packaging is processed by the computer.

By clicking the "save" button, all the images and associated information will be packaged and saved into a local directory or a server. All saved image packages become available for local or remote retrieval. This can be achieved by uploading the files onto a remote server, or by using the default computer as the server with the "Personal Web Server" software from Microsoft, Inc. In the latter case, the saved files should be located directly in, or in subfolders of, the path "c:\webshare\wwwroot\", where "C" is the root drive where the Personal Web Server program is installed. The image files are then available for retrieval by remote computers via a network.

### 4. Security

There are four levels of security for image transfer. First, the retrieval party needs to know the IP address, domain name and the exact path of the image files on the server in order to retrieve the images. This is a lengthy name or a series of numbers or a combination of both. The name(s) of the subfolder(s) can be easily controlled and changed by the server administer, thereby providing the first line of security.

Second, the administrator can move the image files from the designated folders to another location, or change the folder or file names, thus making the folder inaccessible by outside computers. Also, the server computer can be turned off. This will effectively prevent unauthorized user from accessing the files from a remote computer.

Third, the password function is an integrated part of the VT program and can prevent unauthorized users from accessing these image files. Without entering the correct password, the remote computer would not be able to go beyond the first screen even if they have the VT program installed in their computers and know the exact location of these images. The password can be easily changed during image posting. In the event the senders forget the password entered, they can just repost the images with a new password and the old one will be automatically voided as long as the files are saved in the same folder.

Finally the "extra security" function would enhance the security during image storage and transmission.

### 5. Image retrieval

Fig. 2b is a flow chart that explains the image retrieval process. Image retrieval is performed in two steps. First the retrieving computer sends a signal to the server computer to retrieve the names and the thumbnail images in the index file. The users need to enter the correct URL (for remote computers) or file path (for the default computer), and then the correct password in order to have access to this information located in the server computer. This retrieval is achieved via the network by reading the index file compiled by the VT program during image preparation and posting.

The file names and thumbnails, each corresponding to one image file on the server, will be displayed on the remote computer as a virtual slide tray, with up to 20 slide per tray. These thumbnail images and names give the viewers a clear indication of the available images and data for retrieval. This process is completed quickly, as at this stage only the index file and the small thumbnail images are retrieved and displayed, and the relatively large files of images have not been transmitted.

Once this is completed, the remote users can then select the desired slide by clicking on it, and the corresponding image package and related data files will be transferred to the remote computer. Depending on the speed of transmission and the size of the files, this step may take less than a second to several minutes. With broad bandwidth Internet connection (cable, T1, T3, DSL, etc.), this step typically takes less than a few seconds. Once a slide is clicked, the entire background image (the one captured by the high-resolution scanner or the low power objective lens) will be displayed on the screen. At the same time, the linked images and all other linked information for this slide continuously download in the background.

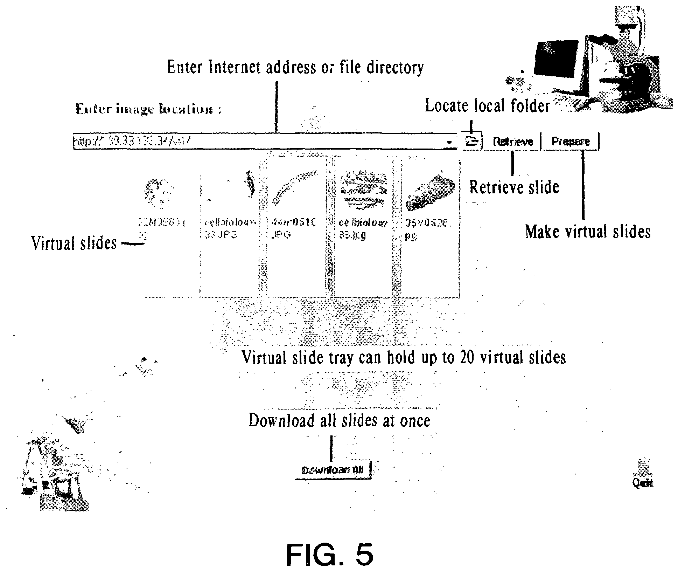

A user may also use the "download all" option. Once this button is clicked, all the available images in the slide tray will be downloaded to the user's computer. This process will take longer than download one slide at a time, but will facilitate a faster retrieval and processing speed in subsequent manipulations of the images. Fig. 5 shows an example screen for the image retrieval and shows five virtual slides for selection. The address of the image location on the Internet or local computer should be entered into the address window. The virtual slides will be retrieved and displayed on the screen on a virtual slide tray. Clicking on any slide will retrieve the virtual slide into the computer. The viewer may also select the "download all" option to download all images into the viewing computer at once.

The retrieval program is compiled with the Lingo language within Director 8.0, using the "GetNetThing" command. This function can also be achieved by using other commands with other computer languages.

### 6. Image manipulation

Once the image is retrieved, it automatically enters into the internal cast of the VT program and is used as an internal cast number. At the same time, the VT program makes smaller versions of the large, high-resolution images. The smaller images are also entered as internal cast numbers. The original images and their smaller counterparts are then used in the VT program and displayed on the "stage" (the entire visible area of the monitor). For this program the stage is an area of "800 x 600" pixels displayed at the center of the monitor, and should occupy the entire screen. We recommend that the computer monitor be set at a resolution of 800 x 600 pixels. It can be higher but not lower.

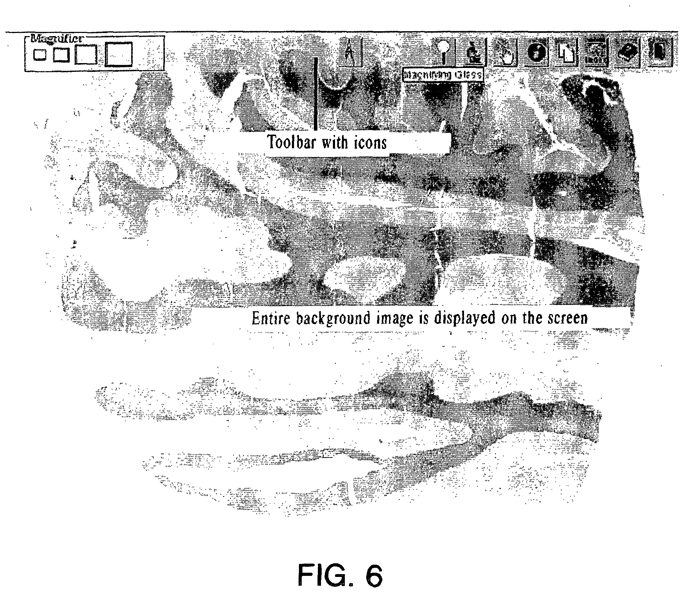

At this stage, the entire image is displayed on the monitor without any obstruction by other images, toolbars or dialogue windows. The toolbar, which is located across the top screen margin, is hidden out of sight automatically. An example tool bar is shown in Fig. 6.

The toolbar becomes visible only when the mouse moves to the upper margin of the screen. The icons in the toolbar are dynamically displayed, i.e. they appear only when the particular functions are relevant and available to the particular screen content. It appears whenever the mouse moves close to the upper border of the screen. Icons for all functions are located in the toolbar. By clicking on the icons in the toolbar, various functions are executed. This automatic hiding of the toolbar serves to maximize the displaying area of the images, ensuring the maximum clarity and the highest magnification possible. The toolbar itself is semitransparent, allowing the image underneath to show through.

Also in the toolbar is a button to call for related patient information and notes. Clicking on the button can display such data on the screen. There is also an "index" button for the users to go back to the virtual slide tray to select another image to exam. Should the users have any question in operating the program, a "help" button is also included in the toolbar. It can be toggled on and off by clicking this button, or the right mouse button from anywhere of the screen. A "tool-tip" will automatically appear when the mouse pauses over an icon for more than one second, providing a simple and clear explanation of the function of the icon in question.

### 7. Magnifying glass effect

There are three modes of image manipulation - two for viewing and one for measurement. The two viewing functions are the "magnifying glass effect" and the "microscope effect". These can be switched on and off by clicking on the corresponding icons in the toolbar. Clicking on the magnifying glass icon in the toolbar turns on this effect.

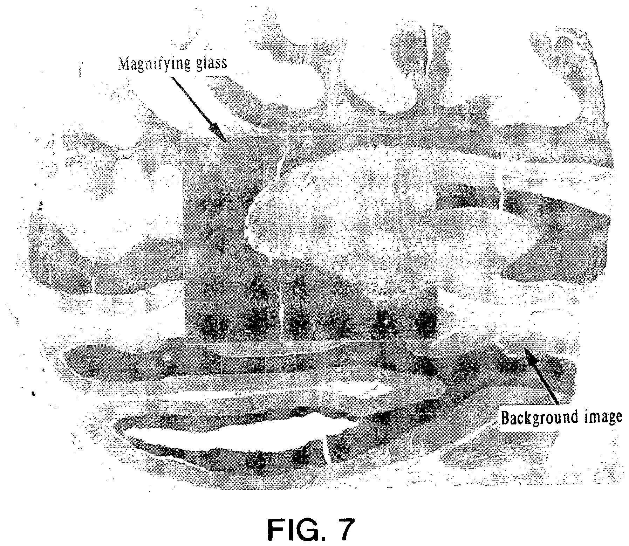

Fig. 7 shows an example screen for the magnifying glass. This may be the default mode and appears on the monitor when the image is first displayed on the screen but the magnifying glass itself is hidden. The "magnifying glass lens" can be moved around the screen with the mouse. Its magnifications can also be changed by clicking the up and down arrows in the toolbar. The enlarged images are always sharp.

The magnifying glass is a rectangle frame of about 300 x 200 pixels in size, and can be turned on and off by left clicking the mouse anywhere on the screen, or clicking on the icon of magnifying glass in the toolbar. The magnifying glass appears at the center of the screen as a rectangular shaped "lens", through which the image within the frame is enlarged by about 1.5-5 times from the background. Both the size of the magnifying glass and the degree of image enlargement within the frame can be adjusted by the users to a certain extent. The "magnifying glass lens" can be dragged around on the screen, allowing enlarged viewing of different areas of the background, in a way similar to viewing a detailed map with a hand hold magnifying glass, only that this magnifying glass lens can adjust its viewing size and magnifications. This function satisfies the need for the users to evaluate any region of the image at low magnifications.

The magnifying glass effect is achieved by using the Lingo language to manage the location and visibility of two layers of images, i.e. the larger version in the back and the identical but smaller version in the front, with another invisible layer called a "mask" in the middle. A mask layer enables the image beneath it to show through only partially from the white area. In this case, the white area has a rectangle shape to give the magnifying glass effect.

In the internal cast file, the large image should be positioned immediately after the "mask" layer. The size of the mask determines the size of the magnifying glass. The size of the large image is larger than the screen size. So when it is fit into the entire screen, the image shrinks in size. The magnifying glass can display a portion of the larger version of the same image located beneath the mask layer and use the center of the mask to dynamically align the foreground and background images to correctly display a changing area within the magnifying glass lens as it is dragged across the screen by the viewer. The size of the large, high-resolution images is used to limit the degree of magnification, so that even at the maximal magnification, the "enlarged" image is not distorted.

### 8. Microscope effect

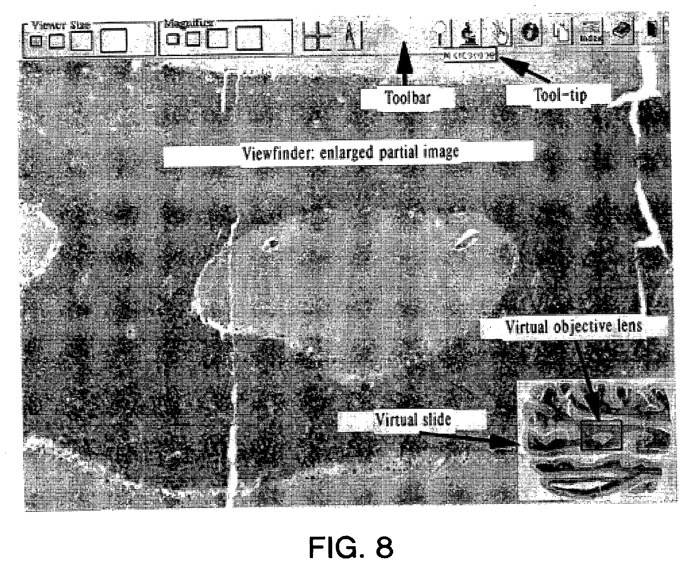

Clicking on the microscope icon in the toolbar turns on the microscope effect. Fig. 8 shows an example screen of the microscope effect. The entire slide is now shrunk to the lower right corner of the screen to serve as the "virtual slide". An "objective lens" within the virtual slide is movable with the mouse.

The area within the small objective lens is enlarged and displayed dynamically on the entire screen. This function closely mimics the operation of a light microscope. This is the main viewing function of the VT program. At this mode, the entire image is displayed at the lower right corner of the screen as a smaller image map. This is the smaller copy of the larger, high-resolution image made by the VT program immediately after image retrieval.

The purpose of making this smaller image is to speed up the image loading process. This smaller image serves as a "virtual slide" containing the entire image, while a portion of the larger, high resolution image is displayed on the entire monitor screen as the viewfinder. Within the virtual slide (the smaller image), there is an even smaller, colored rectangular frame with a 2-pixel border. This frame serves as the objective lens. The image area that is encased inside the "objective lens" is enlarged and displayed on the entire screen.

The size of the objective lens can be changed by clicking on the up and down arrow or the prefixed objective lens size icons in the toolbar. The enlarged image of the area defined by the "objective lens" on the virtual slide fills the entire screen and changes magnifications accordingly. The objective lens can be dragged around within the small image (the virtual slide), and whatever area defined by the objective lens is then displayed instantly on the entire screen.

The smaller image, i.e. the virtual slide, can also be dragged around in the monitor and change sizes. By clicking on the "fixed location icon" in the toolbar, the virtual slide jumps to one of the four corners of the monitor screen, leaving most of the screen for viewing the enlarged area. By clicking on any area outside of the virtual slide, the virtual slide is hidden, leaving the entire monitor free from obstruction for a clear view of the enlarged image of the selected area. The virtual slide can appear anytime by clicking on the left button of the mouse again. This microscopic function, particularly the movement of the objective lens, changing magnifications of the image and the instantaneous displaying of the enlarged images of selective areas on the entire screen, permits further enlargement of the images and closely mimics the manner by which the users evaluate tissue sections on glass slides under a light microscope. When the high-resolution image contains the entire tissue section, this function enables the pathologists to view the entire image at will and offers them the freedom to exam any area of the entire tissue section at a range of magnifications. This affords the users the confidence and ease in operating the system as it functions similar to the traditional microscopic procedure.

The microscope effects are coded by Lingo computer language and image map of the Director 8.0 program. The location, size and movement of the virtual slide, the objective lens and the magnifying glass are continuously traced by identifying the pixel positions of the four-corner coordinates of the images and shapes at all times. In theory, the images can be enlarged indefinitely, but in reality, this is limited by the size of the large image and its ratio to the stage size. The data of the image size is detected at image retrieval, and then compared to the stage size to derive a ratio. This data is then used to restrain the maximum degree of magnification, so that the image would not be enlarged too much to distort the image, thus preserving the truthfulness of image at the highest magnification.

### 9. View linked images and relationship map

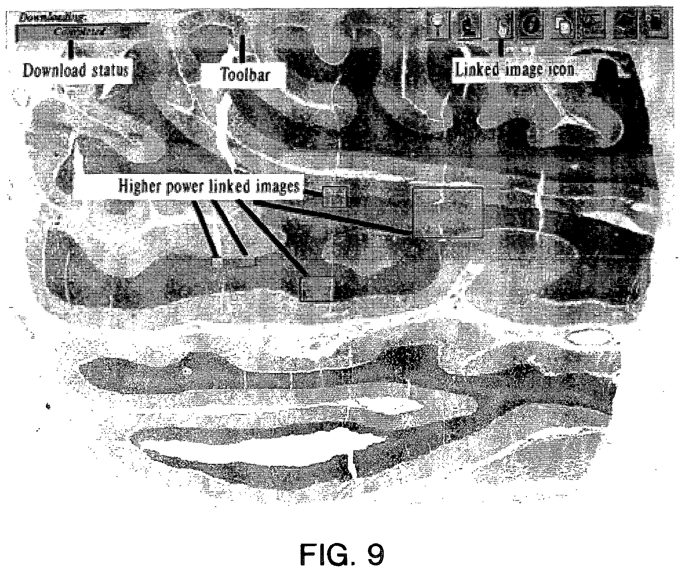

To view the linked higher-power images, one would click the "linked image" button in the toolbar, where it is displayed as a small open hand. The entire background image together with the linked images will be displayed on the screen. Fig. 9 shows an example screen of the linked image map. By selecting the "linked image" button in the toolbar, the viewer can review all the linked higher power images taken from different areas of the same specimen.

The linked image map displays the entire background image and the relative positions and sizes of all linked higher power images. By clicking on a linked image, the entire high power image will be displayed on the screen for close evaluation. The viewer can go back and forth to view different higher power images at will. This screen now displays the entire slide with available linked higher-power images displayed at different locations with different sizes, clearly illustrating the relationship among all the available images from one specimen. The linked images are first displayed as semitransparent yellow rectangle shadows.

Once the downloading of a linked image is completed, the yellow rectangle will have a red outline. This image can then be clicked to display the linked higher-power image on the entire screen. Once a linked image is displayed, functions of all the tools in the toolbar are still available to view the linked images. When finishing viewing this image, one click of the same "linked image" button will bring back the previous screen displaying the image relationship map. One can then select another higher-power image to display.

The feature of the linked image map allows the viewers to evaluate multiple images taken from the same glass slide at different magnifications in a logical and clear process. The entire slide and the linked higher-power images are organized and displayed in a meaningful manner. An example of the layout of the linked images and relationship map is presented in Fig. 4b. When finishing, the viewers can click the "Index" button in the toolbar, and this will bring back the virtual slide tray for the viewers to select another slide to view.

### 10. Image analysis function

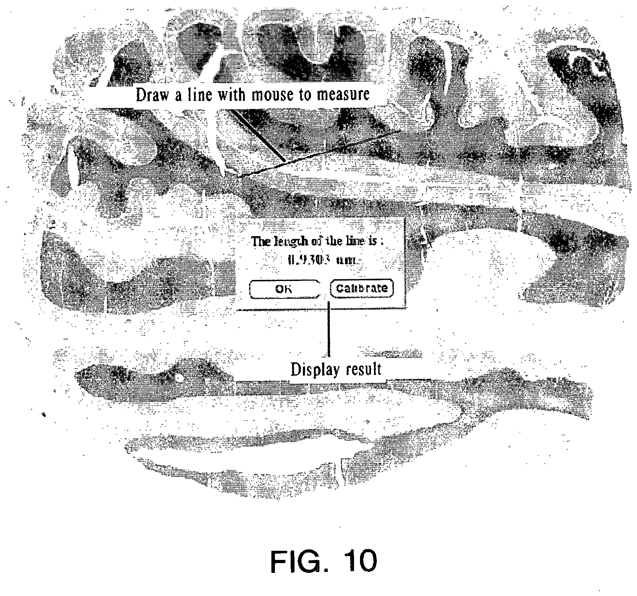

In addition to viewing the images, the VT system enables the users to directly measure the images on the monitor screen. Fig. 10 shows an example screen for the measurement function. By drawing a line between two points on the screen, the VT program will display the correct length of the measurement. When using specified slide scanner and digital camera, the measurement function is pre-calibrated. Otherwise, it needs to be calibrated when measuring each image.

In the measurement mode, the viewer first calibrates the measurement by drawing a line of any length across the screen and then entering its length and the unit. This is a necessary step as the images are captured at different magnifications at a remote site therefore a standardized unit for all cases is not possible. By defining the distance of two points on the screen, the program can establish a measurement unit for the particular image. This length unit is stored in the computer's memory for further measurements until it is reset. This calibration can be performed by drawing a line across the central dimension of a red blood cells or a neutrophil, which in human samples are known to have fixed lengths of about 7.5 um and 15 um respectively.

The calibration can be performed on any other structures on the image with a known distance. The calibration can also be established accurately by measuring particles of standard sizes incorporated into the tissue section, and/or scales engraved or incorporated on the tissue section or the glass slide. Once calibrated, the program will remember the information and use it in all subsequent measurements by recalculating the set value no matter what is the magnification of the image to be measured, until the next calibration is performed.

During calibration, the length and the unit entered by the user is correlated to the pixel information of the line on the screen. This data is then linked to the ratio between the real pixel of the image and the displayed image to take the magnification factor into consideration. The subsequent measurements are performed by multiplying the length of the new line in pixel with the basic unit per pixel established at calibration. This ensures accurate reading of the distance of any two points on the screen at any time at any magnifications of the original image.

This calibration step can be avoided if the user using a scanner with know resolution. The VT program assumes the user employs the recommended high-resolution scanner (Polaroid Sprint4000) with a resolution of 4000x4000 dpi, and has set the default calibration against this enlargement. The program can automatically detect the pixel size of the image, thereby calculate the unit length per pixel. When this scanner is used at this setting, or when a given microscope objective lens is used with fixed resolution, the default calibration will be accurate and used directly without going through the calibration process.

After calibration, to measure the size and distance of any two points on the image at any magnification, the user needs to draw a line with the mouse between any two points on the screen. The correct distance will be calculated and displayed instantly on the screen. Measurement can be repeated indefinitely until the user clicks any other button to exit the measurement mode.

### 11. Image database and instant comparison

The VT system also contains an image database with commonly encountered pathological images. These images can be retrieved at any time and be compared with new images in question. This facilitates accurate and effective diagnostic, consulting and learning experience. The users can also enter their own collection of images for future references.

The reference image database does not have to be stored in the same computer where the user is retrieving images. It can be stored in any remote computer at any location as long as it is connected to the Internet. This allows different hospitals to utilize their specialties in setting up different reference image database for universal comparison and standardization.

### 12. Reporting and feedback function

Once the images are evaluated, the evaluator can communicate with the sender by a number of means, mostly built in the VT program. For example, an email function can be activated within the VT interphase using the computer's default email program. Other functions include videoconferencing, whiteboard image sharing, chat room, Internet phone, etc. These functions enable the sender and the receiver to communicate, and in particular, it allows the receiver to request more images at specified locations and magnifications other than those already provided by the sender.

Some of these functions are achieved through other commercially available software, with "buttons" within the VT program to access these functions. These other functions make the VT program a complete system for the purpose of conducting telepathology and effective exchange of data and information over the networks.

### 13. Other applications

Overall, this system is a new concept using new processes and new technologies to accomplish the task of image transmission and evaluation. High-resolution static images are captured, processed and transmitted, but are viewed in an interactive and dynamic fashion. It is very easy to use, flexible, reliable and accurate. The entire system with this new process is much more affordable than any of the other available systems for the same or similar purpose.

This system can also be used to view high-resolution images of gross pathological specimens. The system would give consulting pathologists or on-call pathologists the freedom to render expert opinion on cases from distant locations. The VT system may be used to transmit and view X-ray and other medical images.

This program may create new image viewing capabilities. For example, the program may provide a bridge between the light and the electron microscopic (EM) images by shrinking and displaying a mosaic of EM images on the screen and reducing the magnification to the light microscopic level. The users can then zoom in to any region with the magnifying glass and microscope effects to examine the ultrastructual details without losing any resolution. This would allow the same preparation to be evaluated at both the light and the electron microscopic levels without changing samples or instruments.

With this system, images of gross specimen, light microscopic images and EM images can be bridged, linked and packaged together. The same specimen can be examined at different levels in a logical way with the linked image function. In addition, the VT program can be used to evaluate any high-resolution, large sized image that needs to be transmitted and viewed dynamically. Such applications may include microscopic examination of computer chips, analysis and close-up viewing of high-resolution satellite images, viewing large maps, measuring distances, and examining detailed photographs of any subject.

A special version of the VT program has been developed to perform morphologic exams for students, residents, etc. In this version, the administrator can set the source where the packaged images are retrieved. A timer can also be set for a time period during which the images will be available for viewing. This information is protected from the students. By clicking on the "start test" button, the timer will start and testing slides will appear on the screen. The students can then select the slides to review and answer relevant questions. When the set time is up, the program will stop and jump to a new screen for the student to exit.

This program has been used at the College of Medicine, University of South Alabama for teaching medical students. The responses have been overwhelmingly positive from the participating students and instructors.

### 14. Software

The VT program was developed using the multimedia programming Director 8.0 Shockwave Studio. It consists of a stage where the visible elements of the interface for the end users are placed. This is controlled by the backstage scores where the command sequences and relationship among different elements are arranged and displayed.

The individual elements (graphics, text, video, sound, scripts and other programming components) are called casts and are stored in cast libraries. Each cast number can be made into sprites and placed on the stage and/or the scores. Each sprite, cast number, frame or movie (the entire program is a collection of movies) can be further controlled by scripts using a programming language called Lingo. These commands instruct the behaviors of the sprites, the cast members, the frames and the movies. The end product of the program is packaged into an executable file called a projector, or a compressed file that can be run within an Internet browser called Shockwave movie. These standalone programs and files can be executed by the end users on any computer to perform the designed functions of the program.

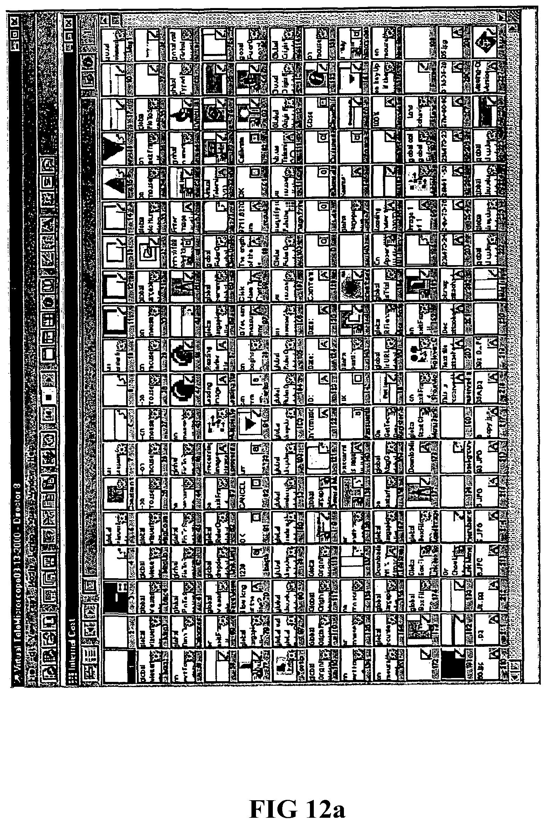

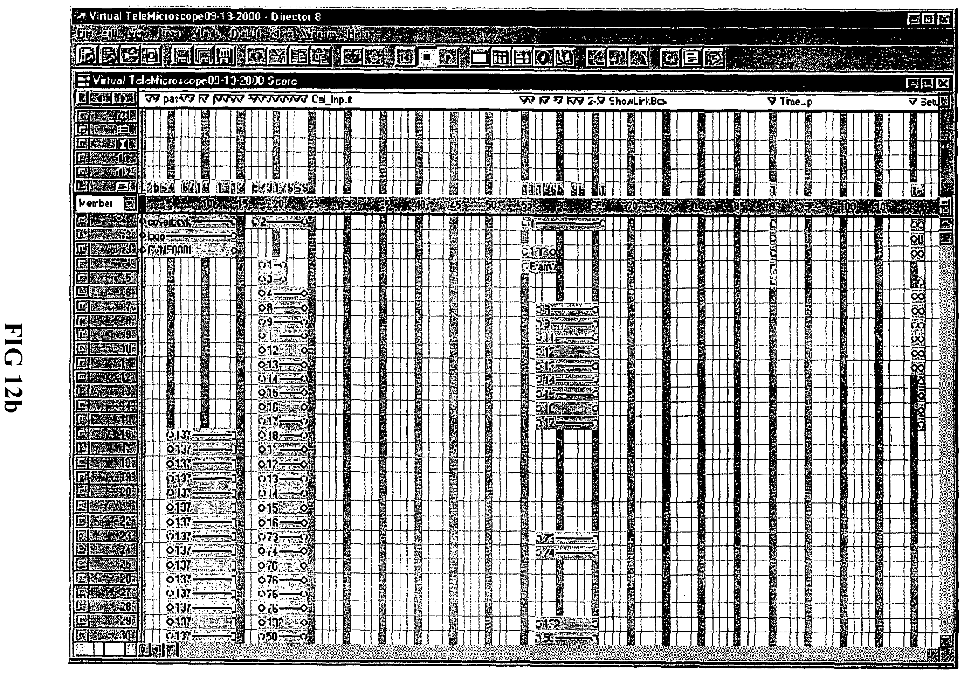

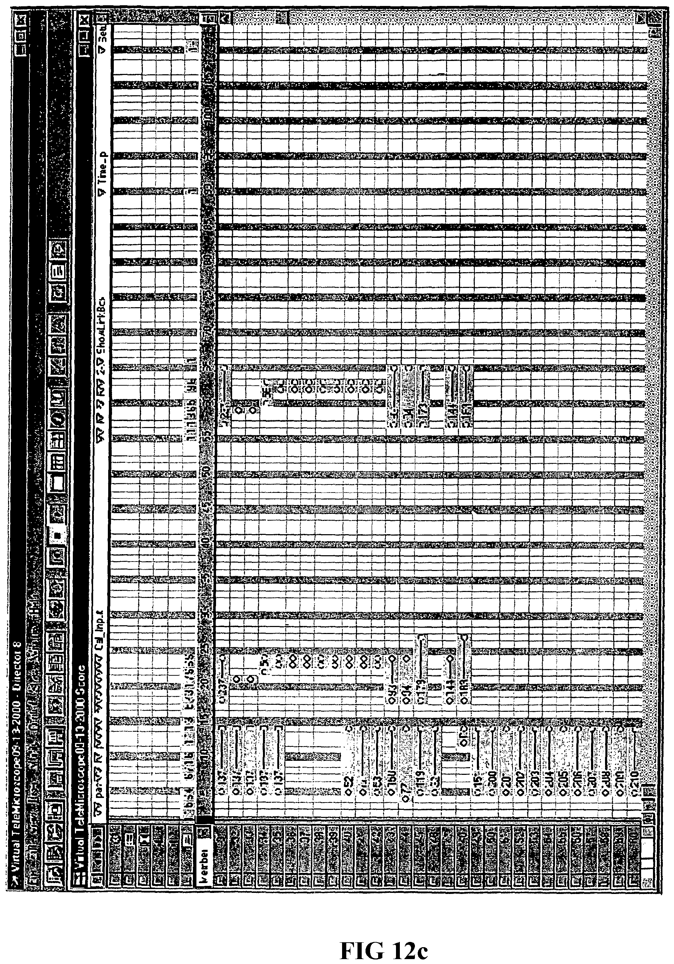

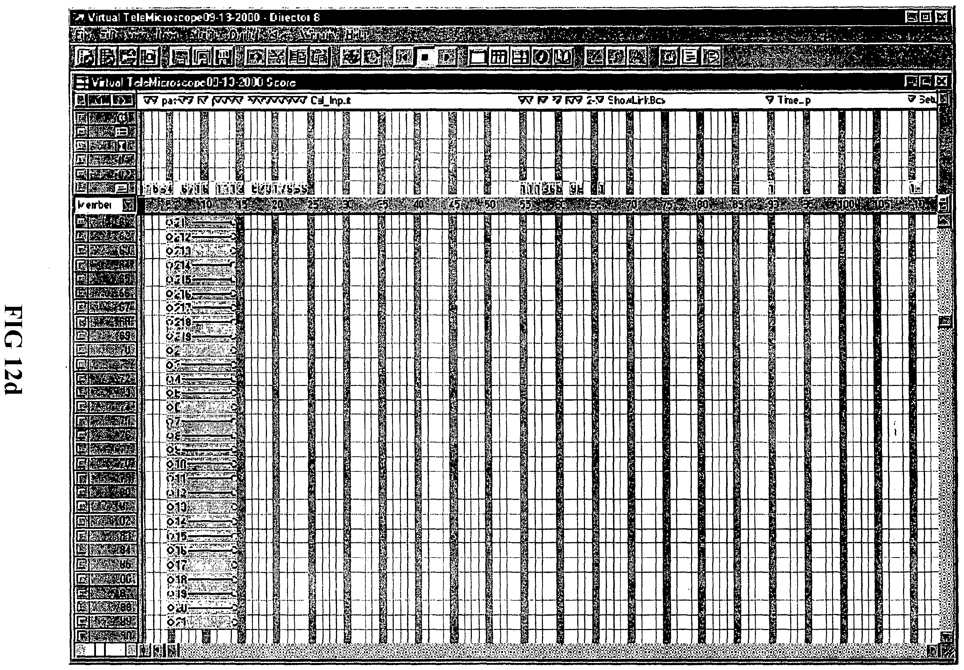

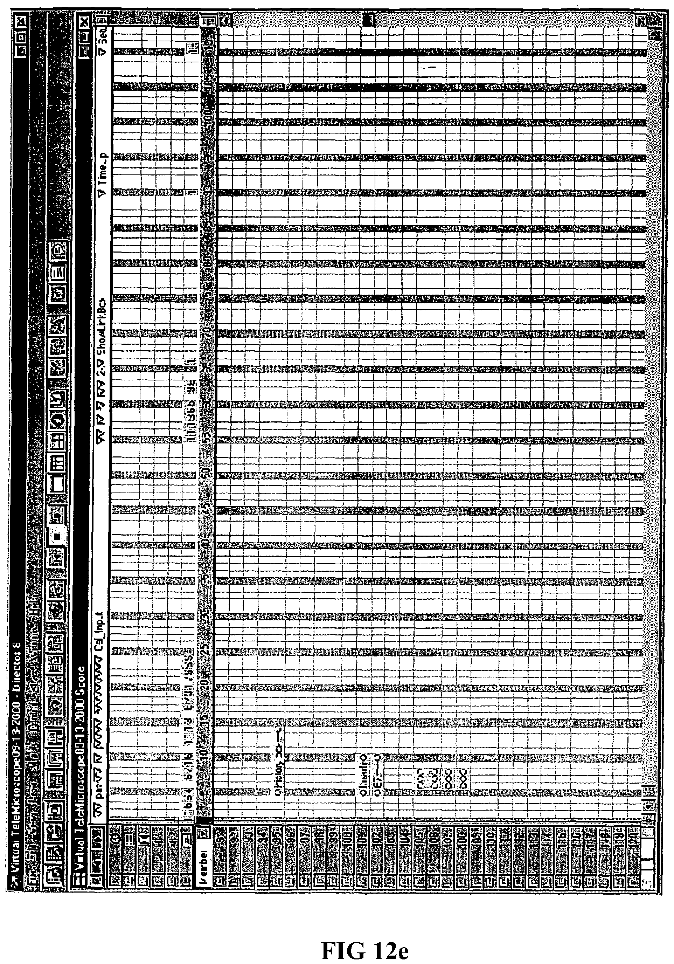

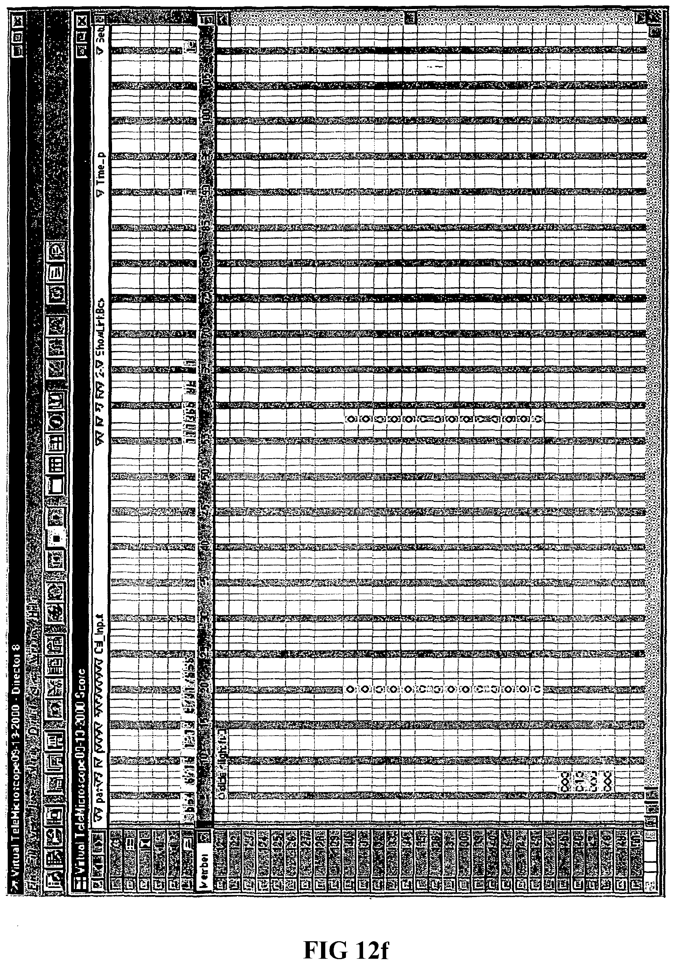

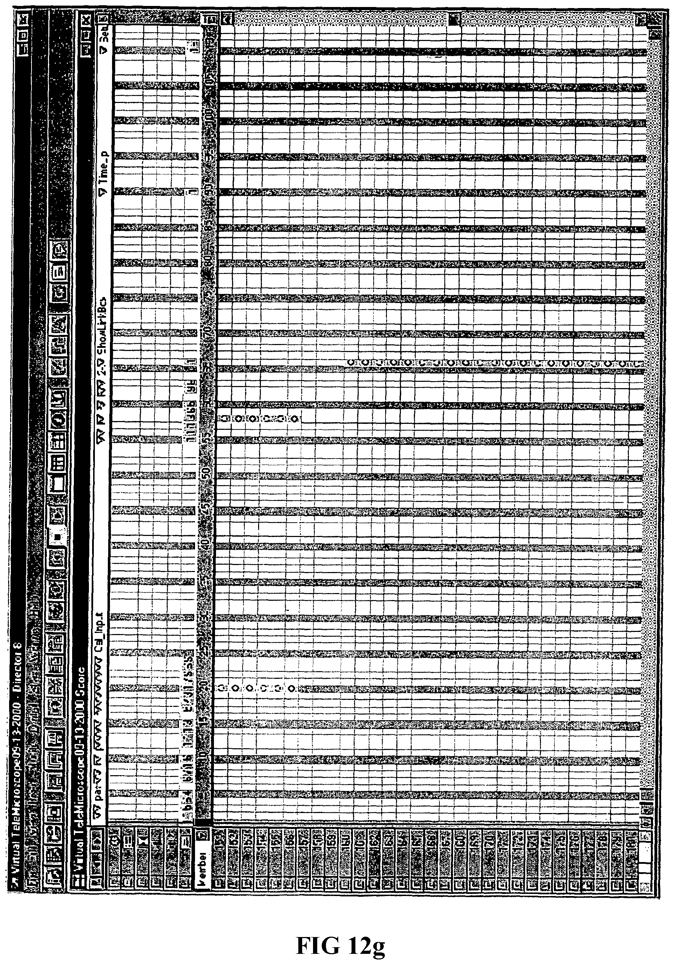

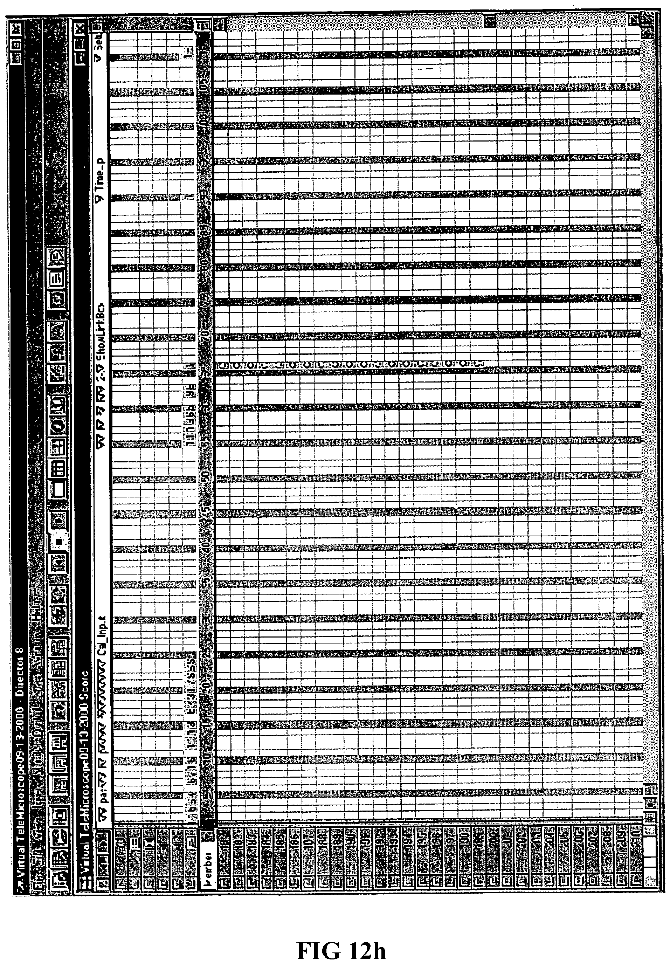

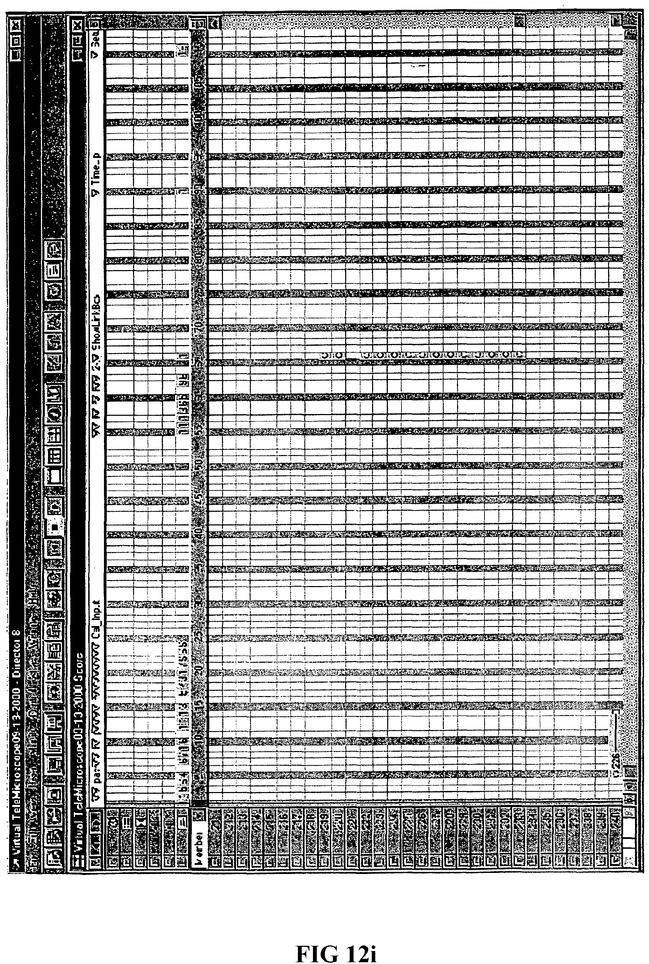

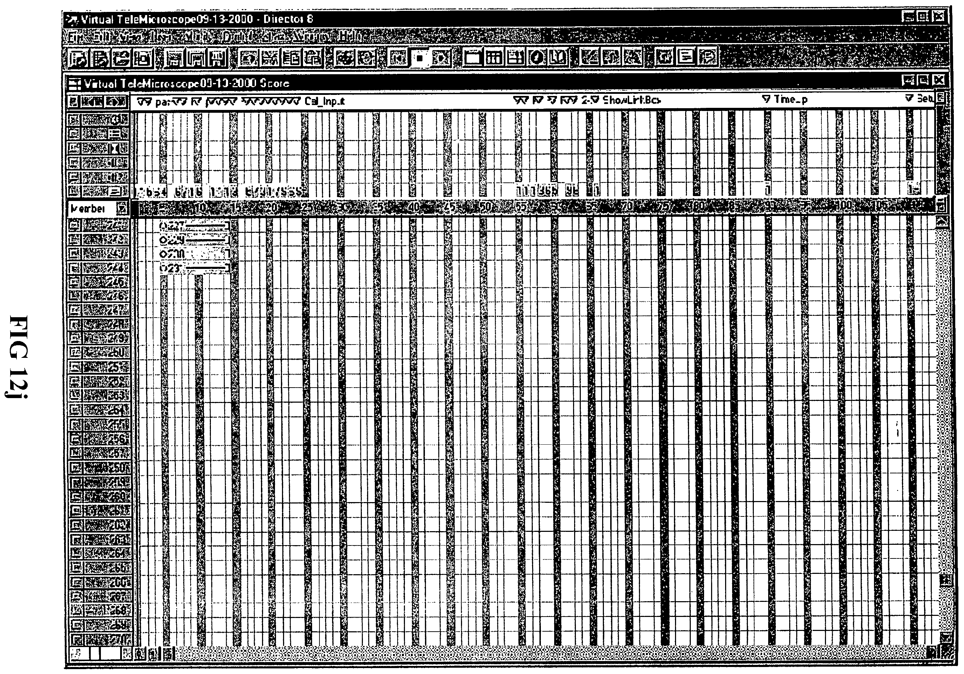

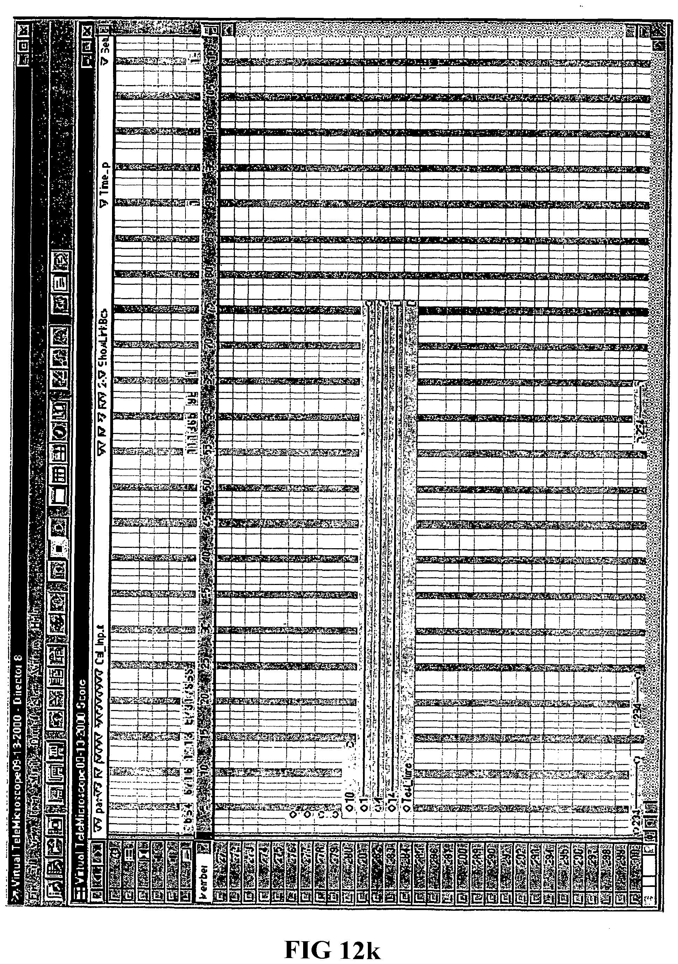

The software code in Appendix A illustrates different aspects of this VT program. Fig. 12a of the drawings is a printout of the internal cast members where most of the casts are stored. Figs. 12b-12k of the drawings are the layout of the backstage scores where the design and arrangement of the sprites are illustrated. Because the score table is very large, it is printed on 10 pages and each page is a continuation from the previous one longitudinally (vertically). The correct sequences of the pages can be identified by the continuity of the channel numbers shown on the left column of the page and they should range from 1 to 300. Pages 2-25 of the Appendix contain a collection of the key script codes written with the Lingo language. Collectively, these documents illustrate the construction of this program.

Although illustrative embodiments of the present invention, and various modifications thereof, have been described in detail herein with reference to the accompanying drawings, it is to be understood that the invention is not limited to these precise embodiments and the described modifications, and that various changes and further modifications may be effected therein by one skilled in the art without departing from the scope of the invention as defined in the appended claims.

### APPENDIX A

### Software for the VT System

Macromedia Director 8.0 and Lingo programming language Index File Retrieval Script

```
 global picNum
 global indexfilecast
 global idxfile
 global thumbfile
 global RFileLoc
 global IsURL
 global RFilename
 global RPath
 global RURLFilename
 global RURLPath
 global TestMode
 global StartClock
 global IndexPath
 on mouseUp
 clearCache
 clearCast()
 ClosePassword()
 set castlib("thumbnail").filename=the moviepath & "empty.cst"
 new(#text,member(1,"indexfile"))
 new(#text,member(4,"temp"))
 new(#text,member(5,"temp"))
 repeat with i=1 to 10
   set the visible of sprite (i+15) to false
   set the visible of sprite (i+25) to false
   set the visible of sprite (i+49) to false
   set the visible of sprite (i+59) to false
   set the visible of sprite (i+69) to false
   set the visible of sprite (i+79) to false
   set the visible of sprite (i+89) to false
 end repeat
 set member(67).text="Reading index file from location. Please
 wait..."
 set the visible of sprite 102 to true
 updatestage
 if (member("url").text starts "http://") or (member("url").text
 starts "ftp://") then
   set IsURL=True
   if the last char of member("url").text <> "/" then
     set member("url").text=member("url").text & "/"
  end if
  AppendHistoryFile
   set tmpLoc=member("url").text
  set ttlchar= the number of chars in tmpLoc
   if char ttlchar-3 to ttlchar of tmpLoc = ".txt" then
     RFileLoc=member("url").text
  else
     RFileLoc=member("url").text & "index.txt" -- Filename is
     assumed as Index.vtm
  end if
  IdentifyURL(RFileLoc)
  set idxfile=preloadnetthing(RFileLoc)
  set thumbfile=preloadnetthing(RURLPath & "thumbnail.cst")
   set IndexPath=RURLPath
   go to "loading"
   if StartClock=True then
     Nothing
   else
      if TestMode then
        StartTimer
        Set StartClock=true
     end if
   end if
 else
   set IsURL=false
   if fileexists(member("url").text)=0 then
     RfileLoc=member("url").text
     IdentifyFilename(RFileLoc)
     AppendHistoryFile
     set IndexPath=rpath
     set castlib("thumbnail").filename=rpath & RemoveExt(RFilename) &
     ".cst"
     importVTM(RfileLoc)
     go to "loading"
     if StartClock=True then
        Nothing
     else
        if TestMode then
          StartTimer
          Set StartClock=true
        end if
     end if
   else
     alert "The location" && member("url").text && "does not contain
     Virtual Telemicroscope Index File. Please verify the location."
     set the visible of sprite 102 to false
     exit
  end if
  end if
  end
  Virtual Slide Selection Script
  global largepicturelist
  global PicSelected
  global indexfilecast
  global currentldl
  global currentPInfo
  global IsURL
  global RFilename
  global RPath
  global RURLFilename
  global RURLPath
  global BoxFilename
  global BoxPointH
  global BoxPointV
  global BoxWidth
  global BoxHeight
  global PreloadComplete
  property spritenum
  on mouseenter
 set the loc of sprite 121= the loc of sprite(spritenum)
 set the visible of sprite 121 to true
 end
 on mouseleave
 set the visible of sprite 121 to false
 end
 on mouseDown
 if sprite(95).visible=false then
   set the visible of sprite 101 to true
   set member("loadingstatus").text="Loading Image..."
   updatestage
   PicSelected=spritenum-15
   GetLinkBoxInfo()
   filedownload()
 end if
 end
 on FileDownload
 new(#text,member(1,"temp"))
 TextFileName=RemoveExt(largepicturelist[PicSelected])
 if IsURL=true then
   set currentldl=preloadnetthing(RURLPath &
   largepicturelist[PicSelected])
   set currentPInfo=preloadnetthing(RURLPath & TextFileName & ".txt")
   go to "preparepic"
 else
   set currentldl=preloadnetthing(largepicturelist[PicSelected])
   set currentPInfo=preloadnetthing(RPath & TextFileName & ".txt")
   go to "preparepic"
 end if
 end
 On GetLinkBoxInfo
 set itemnum=1
 set BoxItem=0
 set BoxCount=1
 BoxFilename=[]
 BoxPointH=[]
 BoxPointV= []
 BoxWidth= []
 BoxHeight=[]
 PreloadComplete=[]
 Repeat with i=1 to member(1,"indexfile").line[PicSelected].char.count
   MyChar=member(1,"indexfile").line[PicSelected].char[i]
        if MyChar="*" then
      set itemnum=itemnum +1
      if itemnum>2 then
        set BoxItem=BoxItem+1
       if BoxItem>5 then
        set tmpBoxFilename=empty
          set tmpBoxPointH=empty
          set tmpBoxPointV=empty
          set tmpBoxWidth=empty
          set tmpBoxHeight=empty
          set BoxItem=1
          set BoxCount=BoxCount+1
       end if
     end if
     next repeat
  else
     if itemnum>2 then
       if BoxItem=1 then
          tmpBoxFilename=tmpBoxFilename & MyChar
          BoxFilename[BoxCount]=tmpBoxFilename
       else if BoxItem=2 then
          tmpBoxPointV= tmpBoxPointV & MyChar
          BoxPointV[BoxCount]=tmpBoxPointV
       else if BoxItem=3 then
          tmpBoxPointH= tmpBoxPointH & MyChar
          BoxPointH[BoxCount]=tmpBoxPointH
       else if BoxItem=4 then
          tmpBoxWidth=tmpBoxWidth & MyChar
          BoxWidth[BoxCount]=tmpBoxWidth
       else if BoxItem=5 then
          tmpBoxHeight=tmpBoxHeight & MyChar
          BoxHeight[BoxCount]=tmpBoxHeight
       end if
    end if
  end if
  end repeat
  end
  -- Index File Loading Script --
  global largepicturelist
  global picNum
  global indexfilecast
  global idxfile
  global thumbfile
  global startingsprite
  global RFileLoc
  global RURLPath
  global IsURL
  global IndexPath
  global PwdCount
  on exitFrame
  set NoThumbnail=false
  set thbstat=getstreamstatus(thumbfile)
  set idxstat=getstreamstatus(idxfile)
  if (getprop(idxstat,#error)="") or (getprop(idxstat,#error)="OK")
  then
  if (not netdone(idxfile)) or (not netdone(thumbfile)) then
    if netdone(idxfile) and (not netdone(thumbfile)) then
        if getprop(thbstat,#bytestotal)<>0 then
        DProg=(float(getprop(thbstat,#bytesSoFar))/float(getprop(thbstat,#bytes
        total)))*100
           set member(67).text="Reading index file... Done" & return &
           "Downloading slides..." && integer(DProg) & "% Completed."
          updatestage
        end if
        -- else
        -- set member(67).text="Reading index file... Done" &
        return & "No slide picture available."
        -- set NoThumbnail=true
        -- end if
     else if (not netdone(idxfile)) and (not netdone(thumbfile)) then
        if getprop(idxstat,#error)="" then
          if getprop(idxstat,#bytestotal)<>0 then
          IProg=(float(getprop(idxstat,#bytesSoFar))/float(getprop(idxstat,#bytes
          total)))*100
             set member(67).text="Reading index file..." &&
             integer(IProg) & "% Completed."
             updatestage
          end if
          go the frame
       end if
     end if
  else if (netdone(idxfile)) and (netdone(thumbfile)) then
     set the visible of sprite 102 to false
     if IsURL=true then
        importfileinto member(4,"temp"),RFileLoc
       if member(4,"temp").type=#empty then
          alert "Index file contains no data or does not exists. Please
          try another location."
          abort
       end if
       identifyURL(RFileLoc)
       if NoThumbnail=false then
          set castlib("thumbnail").filename=RURLPath & "thumbnail.cst"
       else
          -- No thumbnail routine here --
       end if
     else
       nothing
     end if
     if (member(4,"temp").line[member(4,"temp").line.count] contains
     "Password= ") then
       set member(1,"indexfile").text=member(4,"temp").text
     else
       UnProtect(member(4,"temp"),member(1,"indexfile"))
     end if
     updatestage
     indexfilecast=199
     startingsprite=50
     repeat with k=50 to 120
       set the visible of sprite k to false
     end repeat
     largepicturelist=[]
     -- set the filename of castlib "indexfile"=member("url").text
     & "index.cst"
     -- importfileinto member indexfilecast, member("url").text
     & "index.txt"
     if member(1,"indexfile").type=#empty then
       alert "Index file contains no data. Please try another
       location."
       abort
     end if
     picnum=member(1,"indexfile").line.count
     if picNum>20 then
       picnum=20
     else if picnum<=0 then
       set member(67).text="No file found in the location. Please try
       another location."
       set the visible of sprite 102 to true
     else
       set picnum=picnum-1
     end if
     repeat with a=1 to picnum
       set largepicturelist[a]=empty
     end repeat
     -- if
     member(1,"indexfile").line[member(1,"indexfile").line.count].word[2] =
     empty and
     member(1,"indexfile").line[member(1,"indexfile").line.count].word[1] _ =
     "Password=" then
     -- LoadData
     -- go to the frame+1
     if
     member(1,"indexfile").line[member(1,"indexfile").line.count].word[1] =
     "Password=" then
       if
       member(1,"indexfile").line[member(1,"indexfile").line.count].word[2] =
       empty then
          LoadData
          go to the frame+1
       else
          set PwdCount=0
          TypePassword
          go to "pwdwait"
       end if
    else
       alert "Index File Corrupted. Please Try Another Location."
       go to "passed"
    end if
     -- repeat with i=1 to picNum
     -- CharInLine=member(1,"indexfile").line[i].char.count
     -- repeat with j=1 to CharInLine
     -- MyChar=member(1,"indexfile").line[i].char[j]
     -- if MyChar="*" then
     -- new(#text,member(indexfilecast+i))
    -- set member(indexfilecast+i).width=300
     -- set member(indexfilecast+i).boxtype=#fixed
     -- if j=CharInLine then
      -- set
      member(indexfilecast+i).text=largepicturelist[i]
      -- else
      -- set member(indexfilecast+i).text=char j+1 to
      CharInLine of member(1,"indexfile").line[i]
      -- end if
      -- exit repeat
      -- else
      -- largepicturelist[i]=largepicturelist[i] & MyChar
      -- end if
      -- end repeat
      -- set the membernum of sprite(startingsprite+i-1) to
      indexfilecast+i
      -- end repeat
      -- repeat with j=50 to 50+(picnum-1)
      -- set the visible of sprite j to true
      -- set the visible of sprite(j+20) to true
      -- end repeat
      -- updatestage
   end if
 else
   alert "Error downloading file. Please try another location." &
   return & "Error Code: " & getprop(idxstat,#error)
   go to "passed"
   abort
 end if
 go the frame
 end
 -- Switch to Magnifying Glass Effect Script --
 global MagGlass
 global Microscope
 global RulerOn
 on mouseUp
 if MagGlass=true and Microscope=false then
   if RulerOn=true then
      set RulerOn=false
      go to "magnifying"
      set the visible of sprite 3 to true
      set the visible of sprite 4 to true
   else
      if the framelabel="ShowLinkBox" then
        go to "magnifying"
      end if
      if the visible of sprite 3=true then
        set the visible of sprite 3 to false
        set the visible of sprite 4 to false
      else
        set the visible of sprite 3 to true
        set the visible of sprite 4 to true
      end if
   end if
 else
   set MagGlass=true
  set Microscope=False
  go to "magnifying"
  set the visible of sprite 3 to true
  set the visible of sprite 4 to true
  end if
  end
  -- Switch to Microscope Effect Script --
  global viewerheight
  global viewerwidth
  global viewerloc
  global smallpicheight
  global smallpicwidth
  global smallpicloc
  global ruler
  global RulerOn --Check ruler on/off
  global cal
  global dropdown
  global MagGlass
  global Microscope
  global PicToScreenRatio
  on mouseUp
  if MagGlass=false and Microscope=true then
  if RulerOn=True then
     set RulerOn=false
     go to "2nd"
     set the visible of sprite 4 to true
     set the visible of sprite 5 to true
  else
     if the framelabel="ShowLinkBox" then
       go to "2nd"
     end if
     if the visible of sprite 4=true then
       set the visible of sprite 4 to false
       set the visible of sprite 5 to false
     else
       set the visible of sprite 4 to true
       set the visible of sprite 5 to true
     end if
  end if
  else
  set MagGlass=false
  set Microscope=true
  set smallpicheight=member(1).height
  set smallpicwidth=member(1).width
  set smallpicloc=point(233, 189)
  set viewerheight=smallpicheight*0.25
  set viewerwidth=smallpicwidth*0.25
  viewerloc=point(233, 189)
  set dropdown=false
  set the visible of sprite(5) to true
  set the visible of sprite(4) to true
  -- set the height of sprite 5 to viewerheight
  -- set the width of sprite 5 to viewerwidth
  -- set the height of sprite 4 to member(1).height
   -- set the width of sprite 4 to member(1).width
   set the linesize of sprite 5 to 2
   ruler=34 --Ruler spriteNum
   set cal=false
   set RulerOn=false
  go to "2nd"
  GoRightBottom()
  end if
  updatestage
  end
  On GoRightBottom
  if member(2).height<member(2).width then
  set the width of sprite 4 to 192
  else
  set the width of sprite 4 to 148
  end if
  C_ViewerSize()
  set the width of
  sprite(5)=float(sprite(4).width)/((PicToScreenRatio)*2.2)
  C_MagnifierSize()
  sprite(4).rect=rect(800-the width of sprite 4,600-the height of
  sprite 4,800,600)
  updatestage
  end if
  -- Link Images Script --
  global BoxFilename
  global BoxPointH
  global BoxPointV
  global BoxWidth
  global BoxHeight
  global LinkStatus
  global GoToLink
  global HistoryFile
  global MagGlass
  global Microscope
  global LinkHistory
  on mouseUp
  if LinkStatus=False then
  if BoxFilename<>void then
     if BoxFilename.count>0 then
       if the framelabel="ShowLinkBox" then
          ToggleLinkBox(False)
          if getAProp(LinkHistory,#MagGlass)=true then
            go to "Magnifying"
          else
            go to "2nd"
          end if
       else
          set LinkHistory=[:]
          setAProp(LinkHistory,#MagGlass,MagGlass)
          setAProp(LinkHistory,#Microscope,Microscope)
           go to "ShowLinkBox"
           new(#bitmap,member(196))
           new(#bitmap,member(220))
           set member(196).image=member(1).image.duplicate()
           set member(220).image=member(2).image.duplicate()
           AllLinkBoxesOff()
           ShowBoxes()
        end if
      else
        alert "There is no linked image for this image."
      end if
   else
      alert "There is no linked image for this image."
   end if
 else
   set LinkStatus=false
   set GoToLink=True
   erase member(1)
   erase member(2)
   erase member(190)
   erase member(198)
   -- importfileinto member(2) HistoryFile
   -- set the filename of member(190) to HistoryFile
   -- set the filename of member(198) to HistoryFile
   new(#bitmap,member(1))
   new(#bitmap,member(2))
   new(#bitmap,member(190))
   new(#bitmap,member(198))
   set member(1).image=member(196).image.duplicate()
   set member(2).image=member(220).image.duplicate()
   set member(190).image=member(220).image.duplicate()
   set member(198).image=member(220).image.duplicate()
   go to "start"
 end if
 end
 -- Microscope Effect Script --
 on prepareframe
 set the linesize of sprite 5 to 2
 end
 on exitFrame
 if rollover(8) then
   ShowMicmenu()
 else
   HideMicMenu()
 end if
 set the rect of sprite 1 = map(the rect of sprite 4, the rect of
 sprite 5, the rect of sprite 6)
 updatestage
 go the frame
 end
 -- Magnifying Glass Effect (Using Lens Behavior) --
 -- Lens behavior
 -- Info on use is in the behavior description.
 -- For scripters, we're moving the regpoint of the masking graphic
 -- in the opposite direction to that of the drag of the lens.
 -- If the altered graphic is larger than the regular background and
 -- the lens appears to magnify the background, then the mask's
 regPoint is
 -- moved with a scaling factor, and the larger graphic is sent in the
 -- direction opposite to this. (Confusing -- see stepFrame method.)
 -- When the behavior is dragged atop a lens graphic then the routine
 checks
 -- that there is actually a valid masked graphic in a lower channel.
 This check
 -- is performed again at runtime, just in case sprites were moved
 about
 -- after the time that the behavior was assigned.
 -- When the lens is clicked a reference to the behavior is added to
 the actorList.
 -- This is preferable to use of a tight repeat loop because other
 things can still occur
 -- in your movie -- animations can play, sounds or videos can run,
 your email can still arrive.
 -- The stepFrame method checks against the mouse being released and
 will remove itself
 -- from the actorList when the user stops dragging the lens.
 -- Note the Error handler at the end of the set of standard behavior
 routines...
 -- this makes the "FindAndVerifyMasks" routine easier to read, because
 the
 -- handling of an error is separated from the testing of the error.
 -- History:
 -- 10/23/97 jd Added magnification, changed regPoint retention,
 rewrote docs.
 -- 10/22/97 jd Written as D6 behavior.
 -- 6/15/94 jd Hardwired for Director 4.
 global myMagnification
 global PicRatio
 property spriteNum, myOrigLoc, myOrigReg
 global myMaskedLoc, myMask , myMaskedSprite --, myMagnification
 -- EXTERNAL EVENTS
 on beginSprite me
 -- set mymagnification="1"
 FindAndVerifyMasks me
 -- SetMagnifyingFactor me
 -- ResetMaskRegPoint me
 end
 on endSprite me
 ResetMaskRegPoint me
 end
 on mouseDown me
 set myOrigLoc to the loc of sprite spriteNum
 set myOrigReg to the regPoint of member myMask
 set myMaskedLoc to the loc of sprite myMaskedSprite
 add the actorList, me
 end
 on stepFrame me
 if the mouseUp then deleteOne(the actorList, me)
 set newOffset to point(the mouseH, the mouseV) - the clickLoc --
 set the loc of sprite spriteNum to myOrigLoc +
 newOffset*(1+myMagnification*PicRatio)
 set the repoint of member myMask to myOrigReg - myMagnification *
 newOffset
 set the loc of sprite myMaskedSprite to myMaskedLoc -
 (myMagnification -1) * newOffset
 end
 -- SUBROUTINES
 -- Makes sure there's a sprite with a lower channel with a 'mask' ink,
 -- and that the graphic following this image is a proper 1-bit mask.
 -- Also checks that background-transparent ink is applied to the lens.
 -- Called by beginSprite and getPropertyDescriptionList.
 on FindAndVerifyMasks me
 set myMaskedSprite to 0
 repeat with i = the currentSpriteNum down to 1
   if the ink of sprite i = 9 then set myMaskedSprite to i
 end repeat
 if myMaskedSprite = 0 then Error me, #noMaskedSpriteFound
 set maskedMember to the number of the member of sprite myMaskedSprite
 set myMask to member (maskedMember + 1)
 if the type of myMask <> #bitmap then Error me, #noMaskFound
 if the depth of member myMask > 1 then Error me, #incorrectMaskDepth
 if the ink of sprite (the currentSpriteNum) <> 36 then Error me,
 #wrongInkOnLens
 end
 -- Turns the informative string option presented in getPropDesc
 -- to a numeric magnification factor for scaling.
 -- Called by beginSprite.
 on SetMagnifyingFactor me
 delete the last char of myMagnification
 delete the last char of myMagnification
 set myMagnification to value(myMagnification)
 end
 -- Centers the regPoint of the 1-bit masking graphic,
 -- initializing the process. (Changes to member properties persist
 -- through the session, unlike changes to sprite properties.)
 -- Called by beginSprite and endSprite.
 on ResetMaskRegPoint me
 set theH to integer((the right of the rect of member myMask) / 2)
 set theV to integer((the bottom of the rect of member myMask) / 2)
 set the regPoint of member myMask to point(theH, theV)
 end
 -- STANDARD BEHAVIOR ROUTINES
 --on getPropertyDescriptionList me
 -- if the currentSpriteNum <> 0 then FindAndVerifyMasks me
 -- return [#myMagnification: [#comment: "What magnification of channel
 " & myMaskedSprite & " to the background?", #format: #string,
 #range:["1:1", "1.5:1", "2:1", "2.5:1", "3:1"], #default: 1]]
 --end
 on getBehaviorDescription me
 set linel to "This behavior, when applied to a visible 'lens' type of
 graphic, will appear to change the contents of an underlying graphic.
 It does so by manipulating the mask of a mostly-invisible graphic
 between the lens and the background image. You can appear to magnify
 the background by using a larger altered image sandwiched between the
 background and the lens." & RETURN & RETURN
 set line2 to "Examples include being able to drag a background-
 transparent circle around to see a pseudo-filtered version of the
 underlying graphic, or being able to drag a reading bar across a
 graphic in one language to translate it, or being able to look at the
 skeleton of an animal on display, or finding secret messages in a
 picture." & RETURN & RETURN
 set line3 to "To use:" & RETURN
 set line4 to "1) Make your normal background, the altered version,
 and your lens graphics. If magnifying, make the altered version 1.5
 times as large, or 2 times as large, etc." & RETURN
 set line5 to "2) Make a masking graphic that fits precisely inside
 the visible area of your lens. You can use onion skinning to make it,
 or duplicate the lens and fill it with black before deleting non-black
 pixels, or make the mask first and use it to knock out the proper area
 of the lens. The mask defines which area of the altered graphic will be
 seen through the lens." & RETURN
 set line6 to "3) Use 'Transform Bitmap' on the masking graphic to
 turn it to 1-bit color. Place it in the cast immediately following your
 graphic of the altered background. (For instance, if you have an
 animal, its skeleton, and a lens, then put the mask right after the
 skeleton in the cast.)" & RETURN
 set line7 to "4) Select the normal and altered graphics and drag
 them to the Stage. The altered graphic should be precisely atop the
 normal graphic. Give the altered graphic a 'mask' ink. (Result: you
 should see only the shape of the lens altered. If you wish to change
 the relative location of this mask, then change the regPoint of the 1-
 bit graphic in the Paint Window.)" & RETURN
 set line8 to "5) Drag the lens graphic onto the Stage, set its ink
 and desired magnification, and position it right above your masked
 area. It will appear as if your lens is outlining the altered area.".&
 RETURN
 set line9 to "6) Finally assign this behavior to the lens, start the
 movie, and drag the lens around. You should see the altered area of the
 graphic change, as if the lens is really affecting the background." &
 RETURN & RETURN
 set line10 to "Tips:" & RETURN
 set line11 to "-- Dragging smoothness is controlled by the framerate
 of the movie." & RETURN
 set line12 to "-- Large graphics or high colordepths can slow
 dragging." & RETURN
 set line13 to "-- If they drag the lens outside of the graphic
 boundaries they'll see the white of the Paint Window's canvas. You can
 paste the altered graphic against a rectangle of the background color
 in the Paint Window, if speed is not adversely affected." & RETURN
 set line14 to "-- This routine currently relies upon the background,
 the altered graphic, and the lens all sharing the same centerpoint on
 the Stage. It should be possible to set up custom starting points,
 although this can be tricky to visualize while authoring." & RETURN
 return line1 & line2 & line3 & line4 & line5 & line6 & line7 & line8
 & line9 & line10 & line11 & line12 & line13 & line14
 end
 -- Generic error handler.
 -- Mostly called by FindAndVerifyMasks routine.
 on Error me, whatError
 case (whatError) of
  #noMaskedSpriteFound: alert "There needs to be an altered image
  with the 'mask' ink in a channel lower than the lens in channel " & the
  currentSpriteNum & "."
  #noMaskFound, #incorrectMaskDepth: alert "The castmember
  immediately following your altered graphic in channel " &
  myMaskedSprite & " needs to be a 1-bit graphic mask."
  #wrongInkOnLens: alert "The ink for this draggable lens needs to be
  background-transparent."
  end case
  -- This undefined handler will halt operation
  -- and call up the debugger window for you...
  -- you can replace it with "halt" if you wish:
  debug()
  end
  -- Link Images Sub Functions Script --
  Global BoxFilename
  Global BoxLinkFile
  Global RURLPath
  global BoxPointH
  global BoxPointV
  global BoxWidth
  global BoxHeight
  global PreloadComplete
  global IsURL
  On ToggleLinkBox OnOrOff
 repeat with i=1 to BoxFilename.count
    set the visible of sprite (159+i) to OnOrOff
 end repeat
 end
 On LinkPicPreload
 if BoxLinkFile<=BoxFilename.count then
    FileStateInfo=getStreamStatus(RURLPath & BoxFilename[BoxLinkFile])
    if FileStateInfo.state="Complete" then
      set PreloadComplete[BoxLinkFile]=true
      if the framelabel="ShowLinkBox" then
         ShowLinkBoxFrame(BoxLinkFile)
      end if
      set BoxLinkFile=BoxLinkFile +1
      LinkPicPreload()
    else
      set CurrentDLLink=Preloadnetthing(RURLPath &
      BoxFilename[BoxLinkFile])
      set member(185).text="Image" && BoxLinkFile && "of" &&
      BoxFilename.count
   end if
 else
    set member(185).text="Completed"
    set the rect of sprite 231=rect(17,21,172,39)
    abort
 end if
 end
 on streamStatus URL, state, bytesSoFar, bytesTotal
 if state = "Complete" then
    set PreloadComplete[BoxLinkFile]=true
    if the framelabel="ShowLinkBox" then
      ShowLinkBoxFrame(BoxLinkFile)
   end if
   if BoxLinkFile<=BoxFilename.count then
      set BoxLinkFile=BoxLinkFile +1
      set the rect of sprite 231=rect(17,21,17,39)
      LinkPicPreload()
   else
      set PreloadComplete[BoxLinkFile]=false
      -- tellstreamstatus(False)
   end if
   -- else if state="Error" then
   -- alert "An error occur while trying to download " & URL & ". VT
   will skip this file"
 else
   set percentage=float(bytesSoFar)/float(bytesTotal)
   set BarLength=155 * percentage
   set the rect of sprite 231=rect(17,21,17+BarLength,39)
 end if
 end streamStatus
 On AllLinkBoxesOff
 repeat with i=1 to 20
   set the visible of sprite (159+i) to false
   set the visible of sprite (179+i) to false
 end repeat
 end
 On CheckBoxFileStatus TheUrl
 -- tellstremstatus(true)
 FileStateInfo=getStreamStatus(TheUrl)
 if FileStatelnfo.state<>"Complete" then
   alert "Image Not Ready, Please Try Again Later."
   abort
 end if
 end
 On ShowBoxes
 repeat with i=1 to BoxFilename.count
   set the loc of sprite 159+i to
   point(integer(BoxPointH[i]),integer(BoxPointV[i]))
   set the width of sprite 159+i to integer(BoxWidth[i])
   set the height of sprite 159+i to integer(BoxHeight[i])
   if IsURL=true then
      ShowLinkBoxFrame(i)
   else
      ShowAllLinkBoxFrame(i)
   end if
 end repeat
 ToggleLinkBox(True)
 updatestage
 end
 On ShowLinkBoxFrame(WhichBox)
 if WhichBox<=PreloadComplete.count then
   if PreloadComplete[WhichBox]=true then
      set the loc of sprite (179+WhichBox) to
      point(integer(BoxPointH[WhichBox]),integer(BoxPointV[WhichBox]))
      set the width of sprite (179+WhichBox) to
      integer(BoxWidth[WhichBox])
      set the height of sprite (179+WhichBox) to
      integer(BoxHeight[WhichBox])
      set the visible of sprite (179+WhichBox) to true
   end if
 end if
 end
 On ShowAllLinkBoxFrame WhichBox
 set the loc of sprite (179+WhichBox) to
 point(integer(BoxPointH[WhichBox]),integer(BoxPointV[WhichBox]))
 set the width of sprite (179+WhichBox) to integer(BoxWidth[WhichBox])
 set the height of sprite (179+WhichBox) to
 integer(BoxHeight[WhichBox])
 set the visible of sprite (179+WhichBox) to true
 end
 -- Main Program Settings and Related Sub Functions --
 global largepicturelist
 global picNum
 global indexfilecast
 global idxfile
 global startingsprite
 global PicSelected
 global RPath
 global RFilenmae
 global RURLPath
 global RURLFilename
 global IsURL
 global Boxfilename
 global GoToLink
 global MagCalibrated, MicCalibrated, unit, LengthPerPixel
 global Microscope
 global MagGlass
 global IsTrial, IsCDCheck, IsViewer, IsTest
 global CDVerifyString
 global ProgramVersion
 global TestTime
 global PassTrial
 global TypedPass
 global Def_TTime
 global Def_TLocation
 global Def_TPassword
 On Preparemovie
 the editShortCutsEnabled=1
 set IsTrial=False -- Set if program is 30-days
 Trial
 set IsCDCheck=False -- Set if CD-Check is active
 set IsViewer=False -- Set if program is Viewer
 Only version(No Prepare Button)
 set IsTest=False -- Set If Program is Test
 Version/Mode(With clock shows up)
 set CDVerifyString="vt\vt2\vt2.exe" -- Set the CD-Check checking
 file path
 set ProgramVersion=0019 -- Set the program version to
 update Trial Period to 30 days. 9001 special test Version
 if IsTest=True then -- DO NOT EDIT THIS LINE!!!! -
   -- *** Test Version/Mode Declaration *** --
   -- set TestTime=1*3600 -- Test Time
   set PassTrial=3 -- Trial(s) for
   Administrator Setup Password Prompt
   set member(26,"Timer").text=empty -- Clear Administrator Setup
   Password Box
   set TypedPass=empty -- Clear Internal Password
   Variable for Administrator Setup
   set Def_TTime=5 -- Default Test Time
   set Def_TLocation="http://199.33.133.94/vt/" -- Default Test
   Location
  set Def_TPassword="cb" -- Default Password
   Get_TestFile() -- Get Test Setting From
   test.dat
   set member("url").text=empty
   -- *** End Test Vesion Declaration/Initialization *** --
 end if
 -- *** RULER CALIBRATION SETTING *** --
 -- *** Predefined Setting Using Polaroid SprintScan 4000 *** --
 set MagCalibrated=true -- Indicates the system is
 calibrated for Magnifying Glass
 set MicCalibrated=true -- Indicates the system is
 calibrated for Microscope
 set unit="um" -- Default Unit for
 calibration/Ruler
 set LengthPerPixel=30000.0/4596.0 -- Length/pixel : Use length
 divide by pixel. Use dafault Unit to set Unit.
 -- *** END RULER CALIBRATION SETTING *** --
 tellstreamstatus(true)
 set BoxFilename=empty
 set GoToLink=false
 set MagGlass=true
 set Microscope=false
 set castlib("thumbnail").filename=the moviepath & "empty.cst"
 CheckFile()
 set the exitlock to true
 set the rect of the stage to rect(0,0,800,600)
 set the centerstage to true
 -- cachedocverify #always
 set FloatPrecision=0
 set IsURL=False
 set LinkStatus=False
 set GoToLink=False
 ClosePassword()
 if cacheSize()<5000 then
   cacheSize 5000
   end if
   repeat with i=1 to 150
   if i<=21 then
     member(i,"thumbnail").erase()
   end if
   set the visible of sprite i to true
   end repeat
   end
   On RemoveExt filenamestring
   repeat with i=(the number of chars of filenamestring) down to 1
   if char i of filenamestring="." then
     delete char i of filenamestring
     exit repeat
   else
     delete char i of filenamestring
  end if
  end repeat
  set TrueFilename=empty
  repeat with j=(the number of chars of filenamestring) down to 1
   if char j of filenamestring="\" then
     exit repeat
   else.
     TrueFilename=char j of filenamestring & TrueFilename
   end if
 end repeat
 return TrueFilename
 end
 On PopupInfo
 if member(1,"temp").type=#empty then
   set member("ID").text="N/A"
   set member("Date").text="N/A"
   set member("General").text="No Data Available For This Image."
   set member("Comment").text="No Comment Available For This Image."
   set member("Source").text="N/A"
   set member("Pemail").text="N/A"
   set member("PFileAttach").forecolor=8
   else
   set member("ID").text=member(1,"temp").line[1]
   set member("Date").text=member(1,"temp").line[2]
   set member("General").text=member(1,"temp").line[3]
   set member("Comment").text=member(1,"temp").line[4]
   set member("Source").text=member(1,"temp").line[5]
   set member("Pemail").text=member(1,"temp").line[6]
   if member(1,"temp").line[7]=empty then
     set member("PFileAttach").forecolor=8
  else
     set member("PFileAttach").forecolor=255
  end if
  end if
  end
  On ClosePopup
  set member("ID").text=empty
  set member("Date").text=empty
  set member("General").text=empty
  set member("Comment").text=empty
  set member("Source").text=empty
  set member("Pemail").text=empty
  set member("PFileAttach").forecolor=255
  end
  On TypePassword
  set member("url").editable= false
  set the visible of sprite 145 to true
  set the visible of sprite 146 to true
  set the visible of sprite 147 to true
  set the visible of sprite 148 to true
  end
  On ClosePassword
  set member("url").editable= true
  set the visible of sprite 145 to false
  set the visible of sprite 146 to false
  set the visible of sprite 147 to false
  set the visible of sprite 148 to false
  end
  On RemovePath fullpath
  repeat with i=(the number of chars of fullpath) down to 1
  if char i of fullpath="\" then
     exit repeat
  else
     delete char i of fullpath
  end if
  end repeat
  return fullpath
  end
  On LoadData
  repeat with i=1 to picNum
  set tmpFileLoc=empty
  set tmpIndexFileCast=empty
  new(#text,member(indexfilecast+i))
  set member(indexfilecast+i).width=60
  set member(indexfilecast+i).height=80
  -- set member(indexfilecast+i).boxtype=#fixed
  CharInLine=member(1,"indexfile").line[i].char.count
  set itemnum=1
  repeat with j=1 to CharInLine
     MyChar=member(1,"indexfile").line[i].char[j]
     if MyChar="*" then
       set itemnum=itemnum+1
       next repeat
     else
       if itemnum=1 then
          tmpFileLoc=tmpFileLoc & MyChar
          set largepicturelist[i]=tmpFileLoc
       else if itemnum=2 then
          tmpIndexFileCast=tmpIndexFileCast & MyChar
          set member(indexfilecast+i).text=tmpIndexFileCast
          if (member(indexfilecast+i).text=empty) then
            set member(indexfilecast+i).text=largepicturelist[i]
          end if
       else
          exit repeat
       end if
     end if
     -- if itemnum=2 then
     -- new(#text,member(indexfilecast+i))
     -- set member(indexfilecast+i).width=300
     -- set member(indexfilecast+i).boxtype=#fixed
     -- if j=CharInLine then
     -- set
     member(indexfilecast+i).text=largepicturelist[i]
     -- else
     -- set member(indexfilecast+i).text=char j+1 to
     CharInLine of member(1,"indexfile").line[i]
     -- end if
      -- exit repeat
      -- else
      -- exit
      -- end if
      -- else
      -- largepicturelist[i]=largepicturelist[i] & MyChar
      -- end if
   end repeat
   if member(indexfilecast+i).text=empty then
      set member(indexfilecast+i).text=largepicturelist[i]
   end if
   set the membernum of sprite(startingsprite+i-1) to indexfilecast+i
 end repeat
 repeat with j=50 to 50+(picnum-1)
   set the visible of sprite (j-34) to true
   set the visible of sprite j to true
   set the visible of sprite(j+20) to true
 end repeat
 updatestage
 end
 On GetHistoryFile
 new (#text,member(2,"temp"))
 openHistory=new(xtra "fileio")
 if fileexists(the moviepath & "history.dat")=0 then
   openfile(openhistory,the moviepath & "history.dat",1)
   set member(2,"temp").text=readfile(openhistory)
   closefile(openhistory)
 else
   createfile (openhistory,the moviepath & "history.dat")
 end if
 set openhistory=0
 end
 On AppendHistoryFile
 set found=false
 delete member(2,"temp").char[member(2,"temp").char.count+1]
 AppendHistory=new(xtra "fileio")
 if member(2,"temp").text=empty then
   set member(2,"temp").text =member("url").text
 else
   if member(2,"temp").line.count=10 then
     if member(2,"temp").line[10].char.count<>0 then
        set member(2,"temp").line[10]=empty
        delete member(2,"temp").char[member(2,"temp").char.count]
     else
        delete member(2,"temp").char[member(2,"temp").char.count]
     end if
   end if
   repeat with i=1 to member(2,"temp").line.count
     if member(2,"temp").line[i]=member("url").text then
        set found=true
        exit repeat
     end if
   end repeat
   if found=false then
     set member(2,"temp").text= RETURN & member(2,"temp").text
     set member(2,"temp").line[1]=member("url").text
  end if
  end if
  openfile(Appendhistory,the moviepath & "history.dat",0)
  writestring(appendhistory,member(2,"temp").text)
  closefile(appendhistory)
  end
  On HiliteLine LineNum,WhichMember
  if lineNum=1 then
  hilite char 1 to (whichmember.line[1].char.count +1) of field
  WhichMember
  else
  set startchar=length(line 1 to LineNum-1 of WhichMember.text)+2
  set endchar=whichmember.line[LineNum].char.count+startchar
  hilite char startchar to endchar of field whichmember
  end if
  end
  On CheckFile
  repeat with i=1 to the maxinteger
  ChkReqFile=getNthFileNameInFolder(the moviepath,i)
  if ChkReqFile="tms_tool.dll" then
    exit repeat
  else if ChkReqFile=empty then
    alert "A required dll file was not found." & Return & "Please
    reinstall Virtual TeleMicroscope."
    quit
  end if
  end repeat
  end
  On QuitMainMovie
  -- Stuffs to be cleared once exit --
  member(1).erase()
  member(2).erase()
  member(190).erase()
  member(198).erase()
  member(196).erase()
  member(220).erase()
  set castlib("thumbnail").filename=the moviepath & "empty.cst"
  repeat with i=1 to 21
 member(1,"thumbnail").erase()
 end repeat
 quit
 end
 On QuitPrompt
 set QuitMObj=new(xtra "MUI")
 set QuitMInit=[#buttons:#YesNo,#title:"Quit
 Confirmation",#message:"Quit Virtual
 Telemicroscope?",#movable:True,#icon:#caution]
 set QuitMAns=Alert(QuitMObj,QuitMInit)
 case QuitMAns of
  1: QuitMainMovie()
  2: nothing --continue
  otherwise: abort
  end case
  end
  On ClearCast
  set castlib("thumbnail").filename=the moviepath & "empty.cst"
  repeat with i=1 to 21
  member(1,"thumbnail").erase()
  end repeat
  end
  On hideAttachFile
  set the visible of sprite(153) to false
  set the visible of sprite(154) to false
  set the visible of sprite(155) to false
  set the visible of sprite(156) to false
  end
  -- Line Measurement For Calibration and Measuring Script --
  global RulerOn
  global ruler
  global point1
  global point2
  global cal
  global linelength
  global viewerheight
  global smallpicheight
  global LengthPerPixel
  global dropdown
  global RefMag
  global unit
  global PicToScreenRatio
  on mouseDown
  if dropdown=false then
  if RulerOn=false then
    if the visible of sprite 3=true then
       set the visible of sprite 3 to false
       set the visible of sprite 4 to false
    else
       set the visible of sprite 3 to true
       set the visible of sprite 4 to true
    end if
  else
    set the width of sprite(ruler)=1
    set the loc of sprite(ruler)=the mouseloc
    updatestage
    point1=sprite(ruler).loc
    repeat while the stilldown
       point2=the mouseloc
       Hdist=float(point2[2]-point1[2])
       Wdist=float(point2[1]-point1[1])
       if (Hdist>0) and (Wdist>0) then
        angle=float((atan(hdist/wdist)*180)/pi)
        set the rotation of sprite(ruler)=angle
        updatestage
      else if (Hdist<0) and (Wdist>0) then
        angle=float((atan(hdist/wdist)*180)/pi)
        set the rotation of sprite(ruler)=angle
        updatestage
      else if (Hdist<0) and (Wdist<0) then
        angle=180+float((atan(hdist/wdist)*180)/pi)
        set the rotation of sprite(ruler)=angle
        updatestage
      else if (Hdist>0) and (Wdist<0) then
        angle=180+float((atan(hdist/wdist)*180)/pi)
        set the rotation of sprite(ruler)=angle
        updatestage
      end if
      linelength=sqrt(hdist*hdist+wdist*wdist)
      set the width of sprite(ruler)=linelength
     updatestage
   end repeat
 end if
 end if
 end
 on Mouseup
 if dropdown=false then
 if (cal=true) and (RulerOn=true) then
   if linelength>0 then
     go to "2-cal_input"
   else
     alert "Plelse Drag A Line Before Proceeding."
     abort
   end if
 else if (cal=false) and (RulerOn=true) then
   actuallength=linelength*LengthPerPixel*PicToScreenRatio
   set member("result").text=string(actuallength)&&unit
   go to "calibrate"
   updatestage
 end if
 end if
 end
 on rightmouseup
 if the framelabel="help2" then
 go to "Magnifying"
 else
 go to "help2"
 end if
 end
 on endsprite
 repeat with i=1 to 50
 set the visible of sprite i to true
 end repeat
 end
```

## Claims

1. A method of using a computer system as a virtual telemicroscope, comprising the steps of:
capturing a first image corresponding to an entire area of a specimen with a digital image capturing device;
capturing at least one second image corresponding to a selected area of the first image, said at least one second image having a higher magnification than the first image;
storing the first and second images in a computer-readable medium;
generating a linking information map indicating the relationship between said first and second images;
generating a virtual slide with the first and second images and the linking information map;
transmitting the virtual slide from a sender to a receiver at a remote computer through computer network; and
providing communication between the sender and the receiver of the virtual slide, which allows the receiver to request more images at specified locations and magnifications other than those already provided by the sender.

2. The method of claim 1, wherein said capturing of a second image is performed a plurality of times on different selected areas of the specimen, wherein the linking information map links the first image to each of the plurality of second images.

3. The method of claim 2, further comprising choosing a desired area of the first image for obtaining a corresponding magnified second image thereof after said generating of the linking information map.

4. The method of claim 3, wherein the linking information map facilitates the viewing of a desired area of the specimen by providing the appropriate second image linked to said desired area of the first image.

5. The method of claim 4, wherein said first and second images are stored in a similar format, wherein said format is one of JPG, GIF, TIF or BMP.

6. The method of claim 4, wherein said first image is stored in a format chosen from a group consisting of JPG, GIF, TIF and BMP.

7. The method of claim 4, wherein said second image is stored in a format chosen from a group consisting of JPG, GIF, TIF and BMP.

8. The method of claim 5, further comprising storing of the linking information map in said computer-readable medium.

9. The method of claim 8, wherein said computer-readable medium is one of computer hard drive, portable disk or CD.

10. The method of claim 8, wherein said computer-readable medium is a web server.

11. The method of claim 8, wherein the digital image capturing device is a digital camera.

12. The method of claim 8, wherein the digital image capturing device is a scanner.

13. A virtual telemicroscope system, comprising:
a computer system comprising:
(a) a memory unit;
(b) a processing unit in communication with said memory unit, wherein said processing unit is configured to:
i. capture a first image corresponding to an entire area of a specimen with a digital image capturing device;
ii. capture at least one second image corresponding to a selected area of said first image, said second image having a higher magnification than the first image;
iii. store the first and second images in a computer-readable medium;
iv. generate a linking information map indicating the relationship between said first and second images;
v. generate a virtual slide with the first and second images and the linking information map;
vi. transmit the virtual slide from a sender to a receiver at a remote computer through computer network and
vii. provide communication between the sender and the receiver of the virtual slide, which allows the receiver to request more images at specified locations and magnifications other than those already provided by the sender.

14. The system of claim 13, wherein said digital image capturing device is a digital camera.

15. The system of claim 13, wherein said digital image capturing device is a scanner.

16. A computer-readable medium, comprising:
instruction code for capturing a first image corresponding to an entire area of a specimen with a digital image capturing device;
instruction code for capturing at least one second image corresponding to a selected area of the first image, said second image having a higher magnification than the first image;
instruction code for storing the first and second images in a computer-readable medium;
instruction code for generating a linking information map indicating the relationship between said first and second images;
instruction code for generating a virtual slide with the first and second images and the linking information map;
instruction code for transmitting the virtual slide from a sender to a receiver at a remote computer through computer network; and
instruction code for providing communication between a sender and a receiver of the virtual slide, which allows the receiver to request more images at specified locations and magnifications other than those already provided by the sender.

## Patentansprüche

1. Verfahren zur Verwendung eines Computersystems als ein virtuelles Telemikroskop, das die Schritte umfasst:
Erfassen eines ersten Bildes, das einem gesamten Bereich einer Probe entspricht, mit einer digitalen Bilderfassungsvorrichtung;
Erfassen mindestens eines zweiten Bildes, das einem ausgewählten Bereich des ersten Bildes entspricht, wobei das mindestens eine zweite Bild eine höhere Vergrößerung als das erste Bild aufweist;
Speichern der ersten und zweiten Bilder auf einem computerlesbaren Medium;
Erzeugen einer Verbindungsinformationskarte, die die Beziehung zwischen den ersten und zweiten Bildern angibt;
Erzeugen eines virtuellen Objekts mit den ersten und zweiten Bildern und der Verbindungsinformationskarte;
Übermitteln des virtuellen Objekts von einem Sender zu einem Empfänger an einem entfernten Computer über Computer-Netzwerk; und
Bereitstellen von Kommunikation zwischen dem Sender und dem Empfänger des virtuellen Objekts, die es dem Empfänger erlaubt, mehr Bilder an spezifizierten Stellen und mit spezifizierten Vergrößerungen, andere als jene vom Sender bereits bereitgestellten, anzufordern.

2. Verfahren nach Anspruch 1, wobei das Erfassen eines zweiten Bildes mehrere Male auf verschiedenen ausgewählten Bereichen der Probe durchgeführt wird, wobei die Verbindungsinformationskarte das erste Bild mit jedem der Vielzahl von zweiten Bildern verbindet.

3. Verfahren nach Anspruch 2, das ferner ein Auswählen eines gewünschten Bereichs des ersten Bildes zum Erhalten eines entsprechenden vergrößerten zweiten Bildes davon nach dem Erzeugen der Verbindungsinformationskarte umfasst.

4. Verfahren nach Anspruch 3, wobei die Verbindungsinformationskarte die Betrachtung eines gewünschten Bereichs der Probe durch Bereitstellen des geeigneten zweiten Bildes, das mit dem gewünschten Bereich des ersten Bildes verbunden ist, erleichtert.

5. Verfahren nach Anspruch 4, wobei die ersten und zweiten Bilder in einem ähnlichen Format gespeichert werden, wobei das Format JPG, GIF, TIF oder BMP ist.

6. Verfahren nach Anspruch 4, wobei das erste Bild in einem Format gespeichert wird, das aus einer Gruppe ausgewählt ist, die aus JPG, GIF, TIF und BMP besteht.

7. Verfahren nach Anspruch 4, wobei das zweite Bild in einem Format gespeichert wird, das aus einer Gruppe ausgewählt ist, die aus JPG, GIF, TIF und BMP besteht.

8. Verfahren nach Anspruch 5, das ferner ein Speichern der Verbindungsinformationskarte auf dem computerlesbaren Medium umfasst.

9. Verfahren nach Anspruch 8, wobei das computerlesbare Medium eine Computer-Festplatte, eine tragbare Platte oder eine CD ist.

10. Verfahren nach Anspruch 8, wobei das computerlesbare Medium ein Webserver ist.

11. Verfahren nach Anspruch 8, wobei die digitale Bilderfassungsvorrichtung eine digitale Kamera ist.

12. Verfahren nach Anspruch 8, wobei die digitale Bilderfassungsvorrichtung ein Scanner ist.

13. Virtuelles Telemikroskop-System, umfassend:
ein Computer-System, umfassend:
(a) eine Speichereinheit;
(b) eine Verarbeitungseinheit, die mit der Speichereinheit in Kommunikation ist, wobei die Verarbeitungseinheit konfiguriert ist zum:
i. Erfassen eines ersten Bildes, das einem gesamten Bereich einer Probe entspricht, mit einer digitalen Bilderfassungsvorrichtung;
ii. Erfassen mindestens eines zweiten Bildes, das einem ausgewählten Bereich des ersten Bildes entspricht, wobei das zweite Bild eine höhere Vergrößerung als das erste Bild aufweist;
iii. Speichern der ersten und zweiten Bilder auf einem computerlesbaren Medium;
iv. Erzeugen einer Verbindungsinformationskarte, die die Beziehung zwischen den ersten und zweiten Bildern angibt;
v. Erzeugen eines virtuellen Objekts mit den ersten und zweiten Bildern und der Verbindungsinformationskarte;
vi. Übermitteln des virtuellen Objekts von einem Sender zu einem Empfänger an einem entfernten Computer über Computer-Netzwerk und
vii. Bereitstellen von Kommunikation zwischen dem Sender und dem Empfänger des virtuellen Objekts, die es dem Empfänger erlaubt, mehr Bilder an spezifizierten Stellen und mit spezifizierten Vergrößerungen, andere als jene vom Sender bereits bereitgestellten, anzufordern.

14. System nach Anspruch 13, wobei die digitale Bilderfassungsvorrichtung eine digitale Kamera ist.

15. System nach Anspruch 13, wobei die digitale Bilderfassungsvorrichtung ein Scanner ist.

16. Computerlesbares Medium, umfassend:
Befehlscode zum Erfassen eines ersten Bildes, das einem gesamten Bereich einer Probe entspricht, mit einer digitalen Bilderfassungsvorrichtung;
Befehlscode zum Erfassen mindestens eines zweiten Bildes, das einem ausgewählten Bereich des ersten Bildes entspricht, wobei das zweite Bild eine höhere Vergrößerung als das erste Bild aufweist;
Befehlscode zum Speichern der ersten und zweiten Bilder auf einem computerlesbaren Medium;
Befehlscode zum Erzeugen einer Verbindungsinformationskarte, die die Beziehung zwischen den ersten und zweiten Bildern angibt;
Befehlscode zum Erzeugen eines virtuellen Objekts mit den ersten und zweiten Bildern und der Verbindungsinformationskarte;
Befehlscode zum Übermitteln des virtuellen Objekts von einem Sender zu einem Empfänger an einem entfernten Computer über Computer-Netzwerk; und
Befehlscode zum Bereitstellen von Kommunikation zwischen einem Sender und einem Empfänger des virtuellen Objekts, die es dem Empfänger erlaubt, mehr Bilder an spezifizierten Stellen und mit spezifizierten Vergrößerungen, andere als jene vom Sender bereits bereitgestellten, anzufordern.

## Revendications

1. Procédé pour l'utilisation d'un système informatique comme télémicroscope virtuel, comprenant les étapes de:
capture d'une première image correspondant à une aire de surface entière d'un spécimen avec un dispositif de capture d'image numérique;
capture d'au moins une deuxième image correspondant à une aire de surface choisie de la première image, ladite au moins une deuxième image ayant un agrandissement supérieur à la première image;
enregistrement des première et deuxièmes images sur un support déchiffrable par ordinateur;
génération d'une carte d'informations sur des liens, indiquant la relation entre lesdites première et deuxièmes images;
génération d'un cliché virtuel comportant les première et deuxièmes images et la carte d'informations sur les liens;
transmission du cliché virtuel d'un émetteur à un récepteur sur un ordinateur distant par l'intermédiaire d'un réseau informatique; et
fourniture d'une communication entre l'émetteur et le récepteur du cliché virtuel, permettant au récepteur de demander plus d'images sur des endroits spécifiques et des agrandissements autres que ceux déjà procurés par l'émetteur.

2. Procédé selon la revendication 1, dans lequel ladite capture d'une deuxième image est effectuée une pluralité de fois sur différentes aires de surface choisies du spécimen, tandis que la carte d'informations sur des liens relie la première image à chacune parmi la pluralité des deuxièmes images.

3. Procédé selon la revendication 2, comprenant en outre la sélection d'une aire de surface désirée de la première image pour l'obtention d'une deuxième image agrandie correspondante de celle-ci après ladite génération de la carte d'information sur des liens.

4. Procédé selon la revendication 3, dans lequel la carte d'informations sur des liens facilite la visualisation d'une aire de surface désirée du spécimen en procurant la deuxième image appropriée liée à ladite aire de surface désirée de la première image.

5. Procédé selon la revendication 4, dans lequel lesdites première et deuxièmes images sont enregistrées dans un format similaire, tandis que ledit format est un format JPG, GIF, TIF ou BMP.

6. Procédé selon la revendication 4, dans lequel ladite première image est enregistrée dans un format choisi dans un groupe consistant en JPG, GIF, TIF et BMP.

7. Procédé selon la revendication 4, dans lequel ladite deuxième image est enregistrée dans un format choisi dans un groupe consistant en JPG, GIF, TIF et BMP.

8. Procédé selon la revendication 5, comprenant en outre l'enregistrement de la carte d'informations sur des liens sur ledit support déchiffrable par ordinateur.

9. Procédé selon la revendication 8, dans lequel ledit support déchiffrable par ordinateur est l'un parmi, disque dur, disque portable ou CD.

10. Procédé selon la revendication 8, dans lequel ledit support déchiffrable par ordinateur est un serveur internet.

11. Procédé selon la revendication 8, dans lequel le dispositif de capture d'image numérique est une caméra numérique.

12. Procédé selon la revendication 8, dans lequel le dispositif de capture d'image numérique est un scanner.

13. Système de télémicroscope virtuel, comprenant:
un système informatique comprenant:
(a) une unité mémoire;
(b) une unité de traitement en communication avec ladite unité mémoire, tandis que ladite unité de traitement est configurée pour:
i. capturer une première image correspondant à une aire de surface entière d'un spécimen avec un dispositif de capture d'images numériques;
ii. capturer au moins une deuxième image correspondant à une aire de surface choisie de ladite première image, ladite deuxième image ayant un agrandissement supérieur à celui de la première image;
iii. enregistrer les première et deuxièmes images dans un moyen déchiffrable par ordinateur;
iv générer une carte d'informations sur des liens indiquant la relation entre lesdites première et deuxièmes images;
v. générer un cliché virtuel avec les première et deuxièmes images et la carte d'informations sur des liens;
vi. transmettre le cliché virtuel d'un émetteur à un récepteur sur un ordinateur distant par l'intermédiaire d'un réseau informatique; et
vii. fournir une communication entre l'émetteur et le récepteur du cliché virtuel, permettant au récepteur de requérir plus d'images à des endroits spécifiques et des agrandissements autres que ceux déjà procurés par l'émetteur.

14. Système selon la revendication 13, dans lequel ledit dispositif de capture d'images numériques est une caméra numérique.

15. Système selon la revendication 13, dans lequel ledit dispositif de capture d'images numériques est un scanner.

16. Support déchiffrable par ordinateur, comprenant:
un code d'instruction pour la capture d'une première image correspondant à une aire de surface entière d'un spécimen avec un dispositif de capture d'images numériques;
un code d'instruction pour la capture d'au moins une deuxième images correspondant à une aire de surface sélectionnée de la première image, ladite deuxième image ayant un agrandissement supérieur à la première image;
un code d'instruction pour l'enregistrement des première et deuxièmes images sur un support déchiffrable par ordinateur;
un code d'instruction pour la génération d'une carte d'informations sur des liens, indiquant la relation entre lesdites première et deuxièmes images;
un code d'instruction pour la génération d'un cliché virtuel avec les première et deuxièmes images et la carte d'information sur des liens;
un code d'instruction pour la transmission du cliché virtuel d'un émetteur à un récepteur sur un ordinateur distant par l'intermédiaire d'un réseau informatique; et
un code d'instruction pour la fourniture d'une communication entre un émetteur et un récepteur du cliché virtuel, permettant au récepteur de requérir plus d'images à des endroits spécifiques et des agrandissements autres que ceux déjà procurés par l'émetteur.
